# EUROPÄISCHE PATENTSCHRIFT

(11) **EP 4 364 791 B1**
(45) Veröffentlichungstag und Bekanntmachung des Hinweises auf die Patenterteilung: **26.03.2025**
(21) Anmeldenummer: 24165502.6
(22) Anmeldetag: 11.04.2019
(51) Int. Cl.: A61N 1/05, A61N 1/36

(54) **ELEKTRODENANORDNUNG ZUR ELEKTRISCHEN STIMULATION SOWIE VERFAHREN ZUR HERSTELLUNG EINER ELEKTRODENANORDNUNG**
ELECTRODE ASSEMBLY FOR ELECTRICAL STIMULATION AND METHOD FOR PRODUCING AN ELECTRODE ASSEMBLY
ENSEMBLE ÉLECTRODE POUR STIMULATION ÉLECTRIQUE ET PROCÉDÉ DE FABRICATION D'UN ENSEMBLE ÉLECTRODE

(30) Priorität: 27.04.2018 DE 102018110239
(43) Veröffentlichungstag der Anmeldung: 08.05.2024
(62) Teilanmeldung aus: 19718633.1
(73) Patentinhaber: Precisis GmbH, 69124 Heidelberg (DE)
(72) Erfinder: TITTELBACH, Michael, 69126 Heidelberg (DE); LIEDLER, Angela, 82319 Starnberg (DE); REMMERT, Gregor, 69115 Heidelberg (DE); JAGSCHIES, Lasse, 69118 Heidelberg (DE); GÖTTSCHE, Thorsten, 79618 Rheinfelden (DE); GRÄFE, Maik, 79618 Rheinfelden (DE)
(74) Vertreter: Günther, Constantin

(56) Entgegenhaltungen:
- WO-A1-2016/079263
- US-A1- 2017 252 553
- US-B2- 8 774 938

## Beschreibung

Die Erfindung betrifft eine Elektrodenanordnung zur elektrischen Stimulation von Gewebe eines Lebewesens, insbesondere für die Neurostimulation. Des Weiteren betrifft die Erfindung ein Verfahren zur Herstellung einer Elektrodenanordnung zur elektrischen Stimulation von Gewebe eines Lebewesens. Beschrieben wird außerdem ein Elektrodenkörper.

Derartige Elektrodenkörper und Elektrodenanordnungen erzeugen ein elektrisches Feld im Bereich von Gewebe eines Lebewesens zur elektrischen Stimulation dieses Gewebes, wobei die Elektroden durch eine Energiequelle gespeist werden. Die Begriffe Elektrode und Elektrodenkörper sind im Wesentlichen gleichzusetzen, wobei der Begriff Elektrodenkörper nachfolgend insbesondere zur Beschreibung des Aufbaus und der Struktur solcher Elektroden einer Elektrodenanordnung verwendet wird.

EP 2 709 716 A1 zeigt eine Vorrichtung zur Implantation in oder an einem Kopf, wobei die Vorrichtung ein Gehäuse hat, in dem mehrere Elektroden angeordnet sind, wobei die Elektroden ein elektrisches Feld in einer bestimmten Region des Kopfes erzeugen. Die Vorrichtung ist dabei zum Empfangen und Senden von Daten durch W-LAN eingerichtet.

WO 2005/002665 A2 offenbart ein Elektrodensystem zur Implantation in einem Menschen, mit zwei Elektrodenreihen, wobei in einer Elektrodenreihe mehrere Elektroden auf einer Linie nebeneinander angeordnet sind. Die Elektrodenreihen können mit unterschiedlichen Spannungen angesteuert werden, wobei ein elektrisches Feld erzeugt wird.

EP 2 038 004 B1 zeigt ein Elektrodensystem zur Implantierung am Kopf außerhalb des Schädels, mit mehreren Scheibenelektroden, wobei die Scheibenelektroden in einer Matrix angeordnet sind. Dabei können ausgewählte Scheibenelektroden je nach Anwendung durch ein Umschalt-Subsystem angesteuert werden, um ein gewünschtes elektrisches Feld zu erzeugen, ohne die Position der Scheibenelektroden verändern zu müssen. WO 2016/079263 A1, US 8 774 938 B2 und US 2017/252553 A1 zeigen weitere Elektrodensysteme.

Ausgehend hiervon ist es Aufgabe der vorliegenden Erfindung, eine verbesserte Elektrodenanordnung zu schaffen. Weiterhin ist es Aufgabe der vorliegenden Erfindung, ein verbessertes Verfahren zur Herstellung einer Elektrodenanordnung zu schaffen.

Die Aufgabe wird durch eine Elektrodenanordnung mit den Merkmalen des Anspruchs 1 gelöst. Vorteilhafte Ausführungsformen sind in den Unteransprüchen beschrieben.

Dabei kann ein Elektrodenkörper einer Elektrodenanordnung zur elektrischen Stimulation von Gewebe eines Lebewesens, insbesondere für die Neurostimulation vorgesehen. Hierbei ist der Elektrodenkörper dazu eingerichtet sein, an einer Stelle zwischen dem Schädel und der Kopfschwarte eines Lebewesens angeordnet zu werden. Der Elektrodenkörper weist eine Stimulationsoberfläche auf, die dazu eingerichtet ist, in Kontakt mit dem Gewebe des Lebewesens gebracht zu werden, um eine elektrische Stimulation des Gewebes durch Wechselstrom- und/oder Gleichstrom-Pulse zu erzeugen. Hiervon sind auch kombinierte oder überlagerte Wechsel- und Gleichstrompulse umfasst, beispielsweise auch asymmetrische Pulse, die nicht den gleichen momentanen oder integralen Stromfluss in positiver und negativer Richtung aufweisen.

Der Elektrodenkörper kann eine Stimulationsoberfläche mit einem Flächeninhalt von wenigstens 50 mm² haben. Das von dem Elektrodenkörper einer Elektrodenanordnung erzeugte elektrische Feld wird von dem Schädel des Lebewesens abgeschwächt. Indem durch eine wenigstens 50 mm² große Stimulationsoberfläche des Elektrodenkörpers ein ausreichend starkes elektrisches Feld erzeugt wird, ohne die Stimulationsoberfläche durch elektrochemische Prozesse zu beschädigen, kann diese Abschwächung durch den Schädel besser kompensiert werden. Des Weiteren erweist sich die mindestens 50 mm² große Stimulationsoberfläche als vorteilhaft, wenn elektrische Pulse mit einer langen Pulsdauer verwendet werden, da sie bei einer konstanten Stromstärke zu geringeren Stromdichten an der Stimulationsoberfläche führen und somit irreversible elektrochemische Prozesse verringert werden. Überdies wird die potentiell stimulierbare Hirnfläche vergrößert. Die vergleichsweise große Stimulationsoberfläche wirkt sich auch günstig auf das Signal-zu-Rausch-Verhältnis aus.

Alternativ oder zusätzlich kann der Elektrodenkörper eine Stimulationsoberfläche mit einem Flächeninhalt von wenigstens 20 mm² und zusätzlich eine die wirksame Stimulationsoberfläche vergrößernde Oberflächenbehandlung aufweisen. Beispielsweise kann bei einer solchen Oberflächenbehandlung eine rauere oder genoppte Oberfläche oder auch eine fraktale Oberfläche erzeugt werden, wodurch sich eine Oberflächenvergrößerung ergibt und die Übergangsimpedanz zwischen Elektrodenkörper und Gewebe verringert wird, sodass das Signal-zu-Rausch-Verhältnis verbessert wird.

Alternativ oder zusätzlich kann der Elektrodenkörper eine Fixierungsstruktur für die Fixierung des Elektrodenkörpers am Gewebe eines Lebewesens haben.

Alternativ oder zusätzlich kann der Elektrodenkörper einen Leiteranschluss haben, über den der Elektrodenkörper durch Löten, Kleben, Schweißen oder eine anderweitige Verbindungstechnik mit einem elektrischen Leiter zur elektrischen Verbindung des Elektrodenkörpers mit einem elektrischen Gerät oder einem anderen Elektrodenkörper verbunden werden kann.

Alternativ oder zusätzlich kann der Elektrodenkörper bis auf die Stimulationsoberfläche von einem elektrisch isolierenden Trägermaterial vollständig umgeben sein. Dies ermöglicht die einfache Anordnung und Fixierung des Elektrodenkörpers in einer übergeordneten Struktur, insbesondere einer Elektrodenanordnung. Zudem wird durch das elektrisch isolierende Trägermaterial das Auftreten von Leck- und Kurzschlussströmen verhindert oder zumindest reduziert. Hierbei kann der Elektrodenkörper auch von mehreren elektrisch isolierenden Trägermaterialien umgeben sein. Beispielsweise kann auf den Elektrodenkörper ein Polyimid-Substrat aufgebracht sein, das zusätzlich in Silikon eingebettet ist.

Alternativ oder zusätzlich kann der Elektrodenkörper über den Umfang verteilt angeordnete Ausnehmungen zur Fixierung des Elektrodenkörpers in einem Trägermaterial haben. Derartige Ausnehmungen haben den Vorteil, dass der Elektrodenkörper besser in dem Trägermaterial fixiert gehalten werden kann, indem eine verbesserte formschlüssige Verbindung erreicht werden kann. Bei der Herstellung gelangt das zunächst flüssige Trägermaterial wie zum Beispiel ein Silikon in die Ausnehmungen des Elektrodenkörpers, wobei nach Aushärten des Trägermaterials der Elektrodenkörper fest mit dem Trägermaterial verbunden ist. Die Ausnehmungen können zum Beispiel ringförmig um eine Befestigungsöffnung des Elektrodenkörpers und/oder entlang des Elektrodenrands angeordnet sein.

In einer besonders günstigen Ausgestaltung hat das Trägermaterial eine erste zumindest teilweise umlaufende Dichtlippe an einer freiliegenden Kontaktierungsseite des Elektrodenkörpers. Eine umlaufende Dichtlippe hat den Vorteil, dass der Elektrodenkörper vor unerwünschten Wechselwirkungen an der implantierten Stelle mit der Umgebung geschützt wird, wobei die umlaufende Dichtlippe eine Isolierung bereitstellt, die auch bei Deformation des Elektrodenkörpers oder einer den Elektrodenkörper aufnehmenden Elektrodenanordnung intakt bleibt. Im Gegensatz zu aus dem Stand der Technik bekannten Elektrodenkörpern und Elektrodenanordnungen können so die Haltbarkeit verbessert und Kurzschlüsse durch Elektrolyte oder Gewebe, das sich zwischen Elektrode und Schädel befindet, verhindert oder zumindest deutlich reduziert werden. Weiterhin wird das Signal-zu-Rausch-Verhältnis verbessert. Es ist denkbar, dass die erste umlaufende Dichtlippe in einem Stück aus dem Trägermaterial geformt sein kann.

Vorteilhaft ist es, wenn die erste zumindest teilweise umlaufende Dichtlippe den Elektrodenkörper um 0,05 mm bis 1 mm, insbesondere 0,1 mm bis 0,3 mm, überragt. Es hat sich gezeigt, dass diese Abmessungen die Flexibilität des Elektrodenkörpers und einer den Elektrodenkörper aufnehmenden Elektrodenanordnung gewährleisten und gleichzeitig eine effiziente Isolierung des Elektrodenkörpers vor äußeren Einflüssen bereitstellen. Die Dichtlippe kann den Elektrodenkörper dabei in jeder Raumrichtung überragen. Beispielsweise kann die Dichtlippe den Elektrodenkörper in Normalenrichtung der Stimulationsoberfläche und/oder zentripetal zu der Stimulationsoberfläche überragen.

Gemäß einer vorteilhaften Ausführungsform ist der Elektrodenkörper kreisförmig, ellipsenförmig, ringförmig oder bohnenförmig ausgebildet. Mit einer solchen Form ergibt sich eine gute Anlage des Elektrodenkörpers an das Gewebe des Lebewesens.

Günstig ist es, wenn der Leiteranschluss des Elektrodenkörpers als ein tangentialer Leiteranschluss ausgebildet ist. Ein tangentialer Leiteranschluss verläuft dabei orthogonal zur Verbindungslinie zwischen Berührungspunkt und Mittelpunkt des Elektrodenkörpers. Ein über den Leiteranschluss an den Elektrodenkörper anschließbarer Leiter berührt den Elektrodenkörper wie eine Tangente. Dies hat den Vorteil, dass die Verbindungsstelle bei mechanischer Belastung des elektrischen Leiters wie zum Beispiel durch eine Zugkraft entlastet wird, sodass die Wahrscheinlichkeit einer Beschädigung der Verbindungsstelle und damit ein Ausfall der Elektrode verringert wird. Der elektrische Leiter kann dabei zum Beispiel an den Elektrodenkörper angeschweißt oder aufgelötet werden.

Es ist jedoch auch denkbar, dass der Leiteranschluss des Elektrodenkörpers als ein rechtwinkliger Leiteranschluss ausgebildet ist. Unter einem rechtwinkligen Leiteranschluss wird ein Leiteranschluss verstanden, der die Kontur- oder Umfangslinie des Elektrodenkörpers im Anschlussbereich senkrecht schneidet, sodass sich ein rechter Winkel zwischen dem Leiter und der Kontur- oder Umfangslinie im Anschlussbereich ausbildet. Im Vergleich zum tangentialen Leiteranschluss verläuft der rechtwinklige Leiteranschluss parallel statt orthogonal zur Verbindungslinie zwischen Berührungspunkt und Mittelpunkt des Elektrodenkörpers. Diese Leiteranschlussform ist beispielsweise bei Polyimid-Substraten vorteilhaft.

In einer vorteilhaften Ausführungsform ist am Leiteranschluss ein elektrischer Leiter zur elektrischen Verbindung des Elektrodenkörpers mit einem elektrischen Gerät oder einem anderen Elektrodenkörper angeschlossen.

Vorteilhaft ist es, wenn der elektrische Leiter mäanderförmig verläuft. Eine mäanderförmige Anordnung verringert die Belastung des elektrischen Leiters, insbesondere in dessen Anschlussbereich, die zum Beispiel durch Biegen und Ziehen einer den Elektrodenkörper aufnehmenden Elektrodenanordnung entstehen können. Durch die Anordnung des Leiters auf einer gebogenen Bahn wird die Entlastung weiterhin verbessert. Vorteilhafterweise verläuft die gebogene Bahn über einen Winkelbereich von 40° bis 100°, ausgehend von einem 360°-System, um die Primärelektrode.

Gemäß einer günstigen Ausführungsform ist die Verbindungsstelle des elektrischen Leiters mit dem Elektrodenkörper abgedichtet, insbesondere mit einem Epoxidharz abgedichtet. Dies hat den Vorteil, dass die empfindliche Verbindungsstelle vor äußeren Einflüssen wie zum Beispiel Flüssigkeiten geschützt wird, die zu einem Ausfall, zum Beispiel durch Korrosion, der Elektrode führen würde.

In einer vorteilhaften Ausführungsform besteht der elektrische Leiter aus einem Material oder einer Materialzusammensetzung, die in ionenhaltiger Lösung kein elektrochemisches Lokalelement mit dem Material des Elektrodenkörpers bildet. Durch eine solche Materialauswahl wird die Wahrscheinlichkeit korrosiver Prozesse, die zu einer Beschädigung der Elektrode oder des elektrischen Leiters führen, verringert.

Vorteilhafterweise besteht der Elektrodenkörper oder zumindest die Stimulationsoberfläche ganz oder teilweise aus einem biokompatiblen und inerten Werkstoff wie zum Beispiel Platin-Iridium, der, inhärent oder durch eine besondere Beschichtung zum Beispiel mit Iridiumoxid oder Graphen, eine hohe Ladungsinjektionskapazität aufweist.

Vorteilhaft ist es, wenn die Fixierungsstruktur des Elektrodenkörpers eine Isolierstoffhülse aufweist, die zur Isolierung eines mechanischen Befestigungselements gegenüber dem Elektrodenkörper eingerichtet ist. Die Isolierstoffhülse bewirkt somit eine elektrische Isolierung eines mechanischen Befestigungselements wie beispielsweise einer Schraube, sodass keine unerwünschten elektrischen Ströme von dem Elektrodenkörper zu dem Befestigungselement erzeugt werden.

Die Fixierungsstruktur des Elektrodenkörpers kann Fixiermittel an dem Umfang oder einem Ende des Elektrodenkörpers oder auch neben dem oder angrenzend an den Elektrodenkörper in das Trägermaterial eingebettete Fixiermittel aufweisen, um den Elektrodenkörper mit dem Gewebe eines Lebewesens zu verbinden.

In einer günstigen Ausführungsform weist die Fixierungsstruktur wenigstens eine Befestigungsöffnung in dem Elektrodenkörper auf, durch die der Elektrodenkörper durch das mechanische Befestigungselement, z.B. eine Schraube, einen Nagel, eine Klammer, einen Faden oder ein ähnliches mechanisches Befestigungselement, am Gewebe des Lebewesens befestigbar ist. Zudem ist es besonders vorteilhaft, wenn die vorbeschriebene Isolierstoffhülse in der Befestigungsöffnung angeordnet ist. Über die Befestigungsöffnung kann dann der Elektrodenkörper beispielsweise an einer Knochenstruktur des Lebewesens befestigt werden, indem ein mechanisches Befestigungselement, wie zum Beispiel eine Schraube, durch die Befestigungsöffnung, besonders vorteilhaft durch eine in der Befestigungsöffnung angeordnete Isolierstoffhülse, geführt und an der Knochenstruktur fixiert wird. Es sind aber auch andere Befestigungsmittel wie chirurgische Fäden zur Befestigung des Elektrodenkörpers denkbar. In diesem Fall wird die Befestigungsöffnung, vorteilhafterweise auch eine in der Befestigungsöffnung angeordnete Isolierstoffhülse so ausgeführt, dass eine Befestigung des alternativen Befestigungsmittels möglich ist. Beispielsweise weist die Isolierstoffhülse im Fall des chirurgischen Fadens mindestens zwei Öffnungen auf.

Gemäß einer vorteilhaften Ausführungsform hat die Isolierstoffhülse eine zweite zumindest teilweise umlaufende Dichtlippe. Dies hat den Vorteil, dass der Elektrodenkörper gegenüber Flüssigkeiten geschützt wird, die durch Lücken zwischen dem Elektrodenkörper oder der den Elektrodenkörper aufnehmenden Elektrodenanordnung und der Isolierstoffhülse zu dem Elektrodenkörper gelangen können. Es ist denkbar, dass die Isolierstoffhülse oder die zweite umlaufende Dichtlippe in einem Stück aus dem Trägermaterial geformt sein kann.

Vorteilhaft ist es, wenn die Isolierstoffhülse zumindest teilweise aus einem Polyetheretherketon (PEEK) besteht. Polyetheretherketon ist ein hochtemperaturbeständiger thermoplastischer Kunststoff, wobei dieser auch in der Medizintechnik zur Anwendung kommt. Polyetheretherketon hat den Vorteil, dass dieser biokompatibel sowie röntgendurchlässig ist. Zudem hat das Material einen geringen Reibungskoeffizienten, sodass nur eine geringe Kraftübertragung von einem Befestigungsmittel wie einer Schraube auf die Isolierstoffhülse und damit auf den Elektrodenkörper erfolgen kann und diesen damit vor Beschädigung wie zum Beispiel einer Deformation schützt. Polyetheretherketon wirkt auch elektrisch isolierend, sodass keine unerwünschten elektrischen Ströme zwischen dem Elektrodenkörper und dem mechanischen Befestigungsmittel fließen können.

Gemäß einer vorteilhaften Ausgestaltung weist der Elektrodenkörper eine vorgeformte konkave Stimulationsoberfläche auf. Eine solche Stimulationsoberfläche kann sich der äußeren Form der meisten Schädelregionen eines Lebewesens optimal anpassen. Der Elektrodenkörper ist somit besser auf die Krümmung der Knochenstruktur abgestimmt.

Alternativ kann der Elektrodenkörper eine vorgeformte ebene Stimulationsoberfläche mit wenigstens einem Einschnitt und/oder einem Ausschnitt aufweisen, durch den eine flexible Anpassbarkeit des Elektrodenkörpers an die Außenkontur des Schädels eines Lebewesens verbessert ist.

Günstig ist es, wenn der Elektrodenkörper zusätzlich geeignet ist, bioelektrische Signale des Lebewesens zu erfassen. Beispielsweise kann mit Hilfe des Elektrodenkörpers ein Elektroenzephalogramm (EEG) oder Elektrokardiogramm (EKG) oder Elektromyogramm (EMG) aufgenommen werden. Hierzu können ein Verstärker mit Analog- und Digitalwandler (ADC) sowie gegebenenfalls ein analoger oder digitaler Signalfilter mit dem Elektrodenkörper verbunden oder in diesen integriert sein, die im Anschluss an die Erfassung bioelektrischer Signale des Lebewesens zu diagnostischen Zwecken oder zur Steuerung der Stimulation verwendbar sind. Zu diesem Zweck kann überdies eine Auswerte- und Steuereinheit mit dem Elektrodenkörper verbindbar oder in eine an den Elektrodenkörper anschließbare Energiequelle integriert sein.

In einer vorteilhaften Ausführungsform hat der Elektrodenkörper einen Flächeninhalt von maximal 7,1 cm², insbesondere einen Flächeninhalt von maximal 4,6 cm². Ein solcher, vergleichsweise kleingebauter Elektrodenkörper ermöglicht eine gute Integration und einen verringerten Eingriff in das Gewebe des Lebewesens, sodass das Risiko einer Gewebeschädigung verringert ist.

Gemäß einer vorteilhaften Ausführungsform weist der Elektrodenkörper eine Dicke von maximal 2 mm, insbesondere eine Dicke von maximal 1 mm auf. Auf diese Weise kann eine mit einem oder mehreren Elektrodenkörpern gebildete Anordnung ebenfalls eine geringe Dicke, beispielsweise von weniger als 3 mm, weniger als 2 mm oder weniger als 1 mm, aufweisen. Auf diese Weise steht eine implantierte Elektrodenanordnung unter der Kopfhaut des Lebewesens kaum hervor. Dies hat einen kosmetischen Vorteil, wobei die Umwelt die implantierte Elektrodenanordnung nicht wahrnehmen kann. Zudem ist die Wahrscheinlichkeit einer Beschädigung durch äußere Einflüsse verringert, da die potentielle Fläche für derartige Beschädigungen verkleinert ist. Auch wird der Tragekomfort für das Lebewesen durch eine geringere Dicke verbessert.

Somit ist eine Elektrodenanordnung zur elektrischen Stimulation von Gewebe eines Lebewesens, insbesondere eine Elektrodenanordnung für die Neurostimulation vorgesehen, mit wenigstens einer Primärelektrode und wenigstens einer Sekundärelektrode. Hierbei sind eine, mehrere oder alle Primärelektroden jeweils durch einen Elektrodenkörper gebildet und/oder eine, mehrere oder alle Sekundärelektroden sind jeweils durch einen Elektrodenkörper gebildet.

Die wenigstens eine Primärelektrode und die wenigstens eine Sekundärelektrode sind jeweils mittels elektrischer Leiter mit einem Anschlusselement verbunden, wobei das Anschlusselement zum Anschluss an eine andere Elektrode oder eine Energiequelle eingerichtet ist. Alternativ oder zusätzlich sind die Primärelektrode und die Sekundärelektrode an einem gemeinsamen Trägermaterial angeordnet.

Die Elektroden der Elektrodenanordnung sind mit einem Anschlusselement verbunden, wobei das Anschlusselement an eine Energiequelle und/oder an eine elektrische Signalquelle angeschlossen werden kann, um die Elektroden mit Strom zu versorgen. Die elektrische Signalquelle kann zum Senden von elektrischen Stimulationssignalen eingerichtet sein. Die Elektroden können dabei in dem Trägermaterial eingebettet sein. Das Anschlusselement kann dabei einen Steckverbinder aufweisen oder als Steckverbinder ausgebildet sein. Es ist aber auch denkbar, dass das Anschlusselement als Polyetheretherketon-Adapter ausgebildet ist, der den Übergang zwischen der Elektrodenanordnung und der Energiequelle bzw. der elektrischen Signalquelle darstellt. Die Energiequelle und/oder die Signalquelle kann dabei an einer anderen Position im Körper des Lebewesens als die Elektrodenanordnung implantiert werden, wobei die Energiequelle mittels eines elektrischen Leiters mit dem Anschlusselement verbunden und dieser elektrische Leiter mit dem Anschlusselement verschweißt oder verlötet sein kann.

Weiterhin ist eine Elektrodenanordnung zur elektrischen Stimulation von Gewebe eines Lebewesens vorgesehen, insbesondere für die Neurostimulation, wobei die Elektrodenanordnung dazu eingerichtet ist, an einer Stelle zwischen dem Schädel und der Kopfschwarte eines Lebewesens angeordnet zu werden. Die Elektrodenanordnung weist wenigstens eine Primärelektrode und wenigstens eine Sekundärelektrode auf. Sie weist zudem eine Stimulationsoberfläche auf, die dazu eingerichtet ist, in Kontakt mit dem Gewebe des Lebewesens gebracht zu werden, um eine elektrische Stimulation des Gewebes durch Wechselstrom- und/oder Gleichstrom-Pulse zu erzeugen. Hiervon sind auch kombinierte oder überlagerte Wechsel- und Gleichstrompulse umfasst, beispielsweise auch asymmetrische Pulse, die nicht den gleichen momentanen oder integralen Stromfluss in positiver und negativer Richtung aufweisen.

Die erfindungsgemäße Elektrodenanordnung kann dazu eingerichtet sein, zwischen Kopfhaut und Schädel des Lebewesens implantiert zu werden. Gerade im Bereich der Neurostimulation für Epilepsie-Patienten, bei denen eine Behandlung mit Medikamenten nicht anschlägt, wird bisher oft eine maximalinvasive Methode durch Implantation eines intrakraniellen Elektrodensystems angewandt, um den jeweiligen Patienten Linderung zu verschaffen. Die erfindungsgemäße Elektrodenanordnung kann auf dem Schädelknochen des Lebewesens implantiert werden, um die nötige Stimulation bestimmter Gehirnregionen zu erreichen, was einen deutlich weniger invasiven Eingriff bei einem Patienten darstellt und damit eine geringere Belastung für diesen bedeutet.

Die Elektrodenanordnung kann eine Gesamtgröße der Stimulationsoberfläche der Primärelektroden und/oder eine Gesamtgröße der Stimulationsoberfläche aller Sekundärelektroden mit einem Flächeninhalt von wenigstens 50 mm² aufweisen. Das von der Elektrodenanordnung erzeugte elektrische Feld wird von dem Schädel des Lebewesens abgeschwächt. Indem durch eine wenigstens 50 mm² große Stimulationsoberfläche der Elektrodenanordnung ein ausreichend starkes elektrisches Feld erzeugt wird, ohne die Stimulationsoberfläche durch elektrochemische Prozesse zu beschädigen, kann diese Abschwächung durch den Schädel besser kompensiert werden. Des Weiteren erweist sich die mindestens 50 mm² große Stimulationsoberfläche als vorteilhaft, wenn elektrische Pulse mit einer langen Pulsdauer verwendet werden, da sie bei einer konstanten Stromstärke zu geringeren Stromdichten an der Stimulationsoberfläche führen und somit ebenfalls irreversible elektrochemische Prozesse verringert werden. Überdies wird die potentiell stimulierbare Hirnfläche vergrößert. Die vergleichsweise große Stimulationsoberfläche wirkt sich auch günstig auf das Signal-zu-Rausch-Verhältnis aus. Die Elektrodenfläche ist dabei nach oben durch die Gesamtgröße der Elektrodenanordnung begrenzt, um die Elektrodenanordnung beim Menschen noch implantieren zu können.

Alternativ oder zusätzlich kann die Elektrodenanordnung eine Gesamtgröße der Stimulationsoberfläche der Primärelektroden und/oder eine Gesamtgröße der Stimulationsoberfläche aller Sekundärelektroden mit einem Flächeninhalt von wenigstens 20 mm² und zusätzlich eine die wirksame Stimulationsoberfläche vergrößernde Oberflächenbehandlung aufweisen. Beispielsweise kann bei einer solchen Oberflächenbehandlung eine rauere oder genoppte Oberfläche oder auch eine fraktale Oberfläche erzeugt werden, wodurch sich eine Oberflächenvergrößerung ergibt und die Übergangsimpedanz zwischen Elektrodenkörper und Gewebe verringert wird, sodass das Signal-zu-Rausch-Verhältnis verbessert wird.

Alternativ oder zusätzlich weist die Elektrodenanordnung eine Fixierungsstruktur für die Fixierung der Elektrodenanordnung am Gewebe eines Lebewesens auf.

Alternativ oder zusätzlich ist die Elektrodenanordnung bis auf die Stimulationsoberflächen der Primär- und Sekundärelektroden von einem elektrisch isolierenden Trägermaterial vollständig umgeben. Dies ermöglicht die einfache Anordnung und Fixierung der Elektrodenanordnung auf dem Gewebe des Lebewesens. Zudem wird durch das elektrisch isolierende Trägermaterial das Auftreten von Leck- und Kurzschlussströmen an den Elektroden verhindert oder zumindest reduziert. Hierbei kann die Elektrodenanordnung auch von mehreren elektrisch isolierenden Trägermaterialien umgeben sein. Beispielsweise kann auf die Primär- und/oder Sekundärelektroden ein Polyimid-Substrat aufgebracht sein, das zusätzlich in Silikon eingebettet sein kann.

In einer vorteilhaften Ausgestaltung der Elektrodenanordnung ist an dem Trägermaterial ein Verstärkungselement, insbesondere ein Verstärkungsnetz, angeordnet. Durch ein derartiges Verstärkungselement kann die Reißfestigkeit der Elektrodenanordnung erhöht werden. Dies hat den Vorteil, dass die geometrische Genauigkeit der Elektrodenanordnung, d.h. die Lage der Primärelektrode und der Sekundärelektroden zueinander, und damit das notwendige elektrische Feld gewährleistet werden kann. Vorteilhafterweise ist das Verstärkungselement ein Verstärkungsnetz wie zum Beispiel ein chirurgisches Netz, dessen Stränge sich zwischen der Primärelektrode und den Sekundärelektroden erstrecken. Dabei ist es möglich, dass bestimmte Teile der Elektrodenanordnung kein Verstärkungselement haben. So ist zum Beispiel denkbar, dass an dem Kontaktierungsabschnitt der Elektrodenanordnung kein derartiges Verstärkungselement angeordnet ist, um dessen Flexibilität nicht zu verringern.

Gemäß einer vorteilhaften Ausführungsform der Elektrodenanordnung ist zumindest ein elektrischer Leiter zumindest teilweise vom Trägermaterial umgeben. Dies hat den Vorteil, dass der elektrische Leiter an dem Trägermaterial in seiner Position gehalten wird. Dies vermeidet eine unerwünschte Verlagerung des elektrischen Leiters. Zudem wird der elektrische Leiter gegen äußere Einflüsse isoliert, sodass dieser zum Beispiel vor Flüssigkeiten abgeschirmt und damit eine unerwünschte Reaktion verhindert wird.

Günstig ist es, wenn die Dicke der Elektrodenanordnung kleiner als 4 mm, insbesondere kleiner als 2 mm, ist. Auf diese Weise steht eine implantierte Elektrodenanordnung unter der Kopfhaut des Lebewesens kaum hervor. Dies hat einen kosmetischen Vorteil, wobei die Umwelt die implantierte Elektrodenanordnung nicht wahrnehmen kann. Zudem ist die Wahrscheinlichkeit einer Beschädigung durch äußere Einflüsse verringert, da die potentielle Fläche für derartige Beschädigungen verkleinert ist. Auch wird der Tragekomfort für das Lebewesen durch eine geringere Dicke verbessert.

Gemäß einer vorteilhaften Ausführungsform weist die Elektrodenanordnung wenigstens eine Primärelektrode und wenigstens zwei Sekundärelektroden auf. Besonders vorteilhaft ist es, wenn die Elektrodenanordnung mindestens vier Sekundärelektroden aufweist. Eine höhere Anzahl von Elektroden lässt eine genauere Steuerung der elektrischen Feldkonfiguration zu, was zum Beispiel aufgrund von Inhomogenitäten der Leitfähigkeit des Gewebes, oder zur genaueren Fokussierung des elektrischen Feldes auf ein Gewebeareal von Vorteil sein kann. Zudem kann bei mehreren Elektroden zwischen mehreren Kanälen einer Signalableitung gewählt werden, was den Informationsgehalt erhöht. Vorteilhaft ist es, wenn die Elektrodenanordnung zwischen drei und acht Sekundärelektroden hat, insbesondere genau vier Sekundärelektroden.

Vorteilhaft ist es, wenn wenigstens einer der elektrischen Leiter der Sekundärelektroden mäanderförmig um die Primärelektrode angeordnet ist, wobei die mäanderförmige Anordnung des elektrischen Leiters entlang einer gebogenen Bahn um die Primärelektrode verläuft. Eine mäanderförmige Anordnung verringert die mechanische Belastung der elektrischen Leiter, insbesondere deren Anschlussbereiche, die zum Beispiel durch Biegen und Ziehen der Elektrodenanordnung entstehen kann. Durch die Anordnung auf einer gebogenen Bahn wird die Entlastung weiterhin verbessert. Vorteilhafterweise verläuft die gebogene Bahn über einen Winkelbereich von 40° bis 100°, ausgehend von einem 360°-System, um die Primärelektrode.

Grundsätzlich kann das Trägermaterial der Elektrodenanordnung Befestigungsstrukturen aufweisen, mit denen die Elektrodenanordnung an einer Knochenstruktur des Lebewesens befestigbar ist. Die Befestigungsstrukturen können beispielsweise als Schraublöcher oder Ösen zur Herstellung einer Nahtverbindung ausgebildet sein.

Alternativ oder in Kombination haben die Primärelektrode und/oder zumindest eine der Sekundärelektroden jeweils wenigstens eine Befestigungsöffnung, wobei eine Isolierstoffhülse in jeweils einer der Befestigungsöffnungen angeordnet ist. Über die Befestigungsöffnung kann dann die Elektrodenanordnung an einer Gewebestruktur des Lebewesens befestigt werden, indem Befestigungsmittel wie zum Beispiel Schrauben durch die in den Befestigungsöffnungen angeordneten Isolierstoffhülsen geführt werden und an der Gewebestruktur fixiert werden. Es sind aber auch andere Befestigungsmittel wie chirurgische Fäden zur Befestigung der Elektroden denkbar. In diesem Fall wird die Isolierstoffhülse so ausgeführt, dass eine Befestigung des alternativen Befestigungsmittels möglich ist, im Fall des chirurgischen Fadens zum Beispiel durch mindestens zwei Öffnungen in der Isolierstoffhülse. Durch die Festlegung über die Primärelektrode und/oder die Sekundärelektroden können gleichzeitig die übrigen Elektroden an einer gewünschten Position gehalten und ein verbesserter Kontakt zum Schädelknochen erreicht werden, sodass das gewünschte elektrische Feld auch über die Implantationszeit der Elektrodenanordnung an der gewünschten Stelle erzeugt werden kann. Eine derartige Befestigung vermindert zudem unerwünschten Gewebeeinwuchs zwischen Elektrode und Schädelknochen. Weiterhin bewirkt die Isolierstoffhülse eine elektrische Isolierung des Befestigungsmittels, sodass keine unerwünschten elektrischen Ströme von der Primärelektrode und/oder den Sekundärelektroden zu dem Befestigungsmittel erzeugt werden.

Die Isolierstoffhülse kann zumindest teilweise aus einem Polyetheretherketon (PEEK) bestehen. Polyetheretherketon ist ein hochtemperaturbeständiger thermoplastischer Kunststoff, wobei dieser auch in der Medizintechnik zur Anwendung kommt. Polyetheretherketon hat den Vorteil, dass dieser biokompatibel sowie röntgendurchlässig ist. Zudem hat das Material einen geringen Reibungskoeffizienten, sodass nur eine geringe Kraftübertragung von der Schraube auf die Isolierstoffhülse und damit auf die jeweilige Elektrode erfolgen kann und diese damit vor Beschädigung wie zum Beispiel einer Deformation schützt. Polyetheretherketon wirkt auch elektrisch isolierend, sodass keine unerwünschten elektrischen Ströme zwischen den Elektroden und der Schraube fließen können.

Die Isolierstoffhülse der Elektrodenanordnung kann eine zweite zumindest teilweise umlaufende Dichtlippe haben. Dies hat den Vorteil, dass die Elektroden gegenüber Flüssigkeiten, die durch Lücken zwischen der Elektrodenanordnung und der Isolierstoffhülse zu den Elektroden gelangen können, geschützt werden. Es ist denkbar, dass die Isolierstoffhülse oder die zweite umlaufende Dichtlippe in einem Stück aus dem Trägermaterial geformt sein kann.

Alternativ oder in Kombination haben die Primärelektrode und/oder zumindest eine der Sekundärelektroden über den Umfang verteilt angeordnete Ausnehmungen zur Fixierung im Trägermaterial. Derartige Ausnehmungen haben den Vorteil, dass die Primärelektrode und die Sekundärelektroden besser in dem Trägermaterial fixiert gehalten werden können, indem eine verbesserte formschlüssige Verbindung erreicht werden kann. Bei der Herstellung gelangt das zunächst flüssige Trägermaterial wie zum Beispiel ein Silikon in die Ausnehmungen der Primärelektrode und der Sekundärelektroden, wobei nach Aushärten des Trägermaterials die Primärelektrode und die Sekundärelektroden fest mit dem Trägermaterial verbunden sind. Die Ausnehmungen können zum Beispiel ringförmig um die Befestigungsöffnung der Primärelektrode und/oder zumindest einer der Sekundärelektroden und/oder entlang des Elektrodenrands der Primärelektrode und/oder zumindest einer der Sekundärelektroden angeordnet sein.

Alternativ oder in Kombination ist eine Verbindungsstelle des elektrischen Leiters mit der Primärelektrode und/oder mit zumindest einer der Sekundärelektroden ein tangentialer Leiteranschluss. Ein tangentialer Leiteranschluss verläuft dabei orthogonal zur Verbindungslinie zwischen Berührungspunkt und Mittelpunkt der jeweiligen Elektrode. Der Leiter berührt die Elektrode wie eine Tangente. Dies hat den Vorteil, dass die Verbindungsstelle bei Belastung des elektrischen Leiters wie zum Beispiel durch eine Zugkraft entlastet wird, sodass die Wahrscheinlichkeit einer Beschädigung der Verbindungsstelle und damit eines Ausfalls der jeweiligen Elektrode verringert wird. Der elektrische Leiter kann dabei zum Beispiel an die Elektrode angeschweißt oder aufgelötet werden.

Es ist jedoch auch denkbar, dass eine Verbindungsstelle des elektrischen Leiters mit der Primärelektrode und/oder mit zumindest einer der Sekundärelektroden als ein rechtwinkliger Leiteranschluss ausgebildet ist. Unter einem rechtwinkligen Leiteranschluss wird ein Leiteranschluss verstanden, der die Kontur- oder Umfangslinie der Elektrode im Anschlussbereich senkrecht schneidet, sodass sich ein rechter Winkel zwischen dem Leiter und der Kontur- oder Umfangslinie im Anschlussbereich ausbildet. Im Vergleich zum tangentialen Leiteranschluss verläuft der rechtwinklige Leiteranschluss parallel statt orthogonal zur Verbindungslinie zwischen Berührungspunkt und Mittelpunkt der Elektrode. Diese Leiteranschlussform ist beispielsweise bei Polyimid-Substraten vorteilhaft.

In einer günstigen Ausführungsform hat die Elektrodenanordnung einen Kontaktierungsabschnitt, wobei das Anschlusselement an dem Kontaktierungsabschnitt angeordnet ist und wobei der Kontaktierungsabschnitt seitlich von der Elektrodenanordnung abragt. Durch den abragenden Kontaktierungsabschnitt kann das Anschlusselement aus dem Bereich der Primärelektrode und der Sekundärelektrode mechanisch isoliert werden, sodass unerwünschte Wechselwirkungen vermieden werden.

Ferner ist es vorteilhaft, wenn der Kontaktierungsabschnitt eine größere Länge als Breite hat. Die Länge ist die Dimension in Abragerichtung des Kontaktierungsabschnittes, wobei die Breite die Dimension quer zur Abragerichtung des Kontaktierungsabschnittes ist. Durch die längliche Ausgestaltung können auf den Kontaktierungsabschnitt einwirkende Kräfte abgetragen werden, sodass diese Kräfte nicht in den Bereich der Elektroden gelangen und dort zu Beschädigungen führen können.

Weiter ist es vorteilhaft, wenn an dem Kontaktierungsabschnitt Befestigungselemente zur Schraubbefestigung angeordnet sind. Die Befestigungselemente können zum Beispiel als Langlöcher ausgebildet sein, wobei der Kontaktierungsabschnitt mittels Schrauben oder anderer Befestigungsmittel an dem Lebewesen befestigt werden kann. Auf diese Weise kann eine sichere Befestigung des Kontaktierungsabschnittes an dem Lebewesen erfolgen, wobei mechanische Kräfte über den Schädel abgetragen werden können. Die Befestigungselemente können derartig angeordnet sein, dass diese ein sechswertiges Lager bilden, wobei die Rotations- und Translationsfreiheitsgrade beschränkt werden.

Gemäß einer günstigen Ausführungsform ist die Gesamtfläche der Sekundärelektroden größer als die Fläche der Primärelektrode. Diese Ausführungsform ermöglicht eine gute Stromverteilung über die Gewebefläche des Lebewesens und ein verringertes Risiko einer Gewebeschädigung. Ferner ist es vorteilhaft, wenn die Primärelektrode und/oder die Sekundärelektroden jeweils eine Fläche von 50 mm² bis 71 mm², insbesondere eine Fläche von 20 mm² bis 46 mm² haben. Mit diesen Elektrodenflächen wird eine besonders gute Stromverteilung bei verringertem Risiko einer Gewebeschädigung erzielt.

Alternativ kann die Gesamtfläche der Sekundärelektroden in einer vorteilhaften Ausgestaltung auch kleiner sein als die Fläche der Primärelektrode. Diese Ausgestaltung erlaubt eine umgekehrte Stimulationsstromrichtung wie eine anodale Stimulation statt einer kathodalen Stimulation, ohne zu einer korrosionsbedingten Schädigung der Elektroden zu führen. Hierdurch können unterschiedliche Indikationen und Krankheitsbilder therapiert werden.

Vorteilhaft ist es, wenn die Sekundärelektroden auf wenigstens einer Kreisbahn um die Primärelektrode angeordnet sind. In einer weiter vorteilhaften Ausgestaltung können die Sekundärelektroden der Elektrodenanordnung äquidistant auf wenigstens einer Kreisbahn um die Primärelektrode angeordnet sein. Zudem ist es günstig, wenn die Sekundärelektroden auf einer konzentrischen Kreisbahn um die Primärelektrode verteilt sind. Diese Verteilung der Sekundärelektroden um die Primärelektrode begünstigt die Generierung eines elektrischen Feldes durch eine Abschirmung wie zum Beispiel eine Schädeldecke hindurch zusätzlich.

Gemäß einer günstigen Ausführungsform ist der Mittelpunkt wenigstens einer der Sekundärelektroden in einem Abstand von 5 mm bis 55 mm, insbesondere in einem Abstand von 10 mm bis 50 mm, zum Mittelpunkt der Primärelektrode angeordnet. Ferner ist es vorteilhaft, wenn der Rand wenigstens einer der Sekundärelektroden in einem Abstand von 1 mm bis 8 mm, insbesondere in einem Abstand von 2 mm bis 6 mm, zum Rand der Primärelektrode angeordnet ist. Es hat sich gezeigt, dass geringe Abstände der Sekundärelektroden zu der Primärelektrode eine optimale Fokussierung des elektrischen Feldes ermöglichen, wobei durch einen ausreichenden Abstand der Sekundärelektroden zu der Primärelektrode die Wahrscheinlichkeit eines Leck- und Kurzschlussstroms verringert bzw. ein solcher verhindert sowie die Eindringtiefe des elektrischen Feldes in den Kopf vergrößert werden kann. Diese Abstände bilden damit einen optimierten Mittelweg zwischen den gegenläufigen Anforderungen der Fokussierung und der Leck- bzw. Kurzschlussströme. Ein größerer Abstand der Elektroden führt zu einem höheren Signal, aber auch zu einer geringeren Fokalität.

Vorteilhafterweise hat das Trägermaterial wenigstens eine Aussparung, wobei die Aussparung jeweils zwischen benachbarten Sekundärelektroden angeordnet ist. Es ist auch denkbar, dass das Trägermaterial abschnittsweise eine Verjüngung, also eine abnehmende oder geringere Materialstärke aufweist. Auf diese Weise kann die Elektrodenanordnung flexibel ausgestaltet sein. Die Aussparung oder Verjüngung erhöht die Flexibilität insbesondere an den Sekundärelektroden, wobei vorteilhafterweise zwischen jeweils zwei benachbarten Sekundärelektroden eine Aussparung oder Verjüngung angeordnet ist. Durch die erhöhte Flexibilität kann so auf die unregelmäßige Oberfläche der Schädeldecke eines Lebewesens reagiert werden, indem die Elektrodenanordnung an jeder Position einen ausreichenden Kontakt zur Schädeldecke hat.

Es wird vorgeschlagen, dass das Trägermaterial eine erste zumindest teilweise umlaufende Dichtlippe an einer freiliegenden Kontaktierungsseite der Primärelektrode und/oder an zumindest einer der Sekundärelektroden hat. Eine umlaufende Dichtlippe hat den Vorteil, dass die Elektroden vor unerwünschten Wechselwirkungen an der implantierten Stelle mit der Umgebung geschützt werden, wobei die umlaufende Dichtlippe eine Isolierung bereitstellt, die auch bei Deformation der Elektrodenanordnung intakt bleibt. Im Gegensatz zu aus dem Stand der Technik bekannten Elektrodenanordnungen kann so die Haltbarkeit verbessert sowie Kurzschlüsse durch Elektrolyte oder Gewebe, das sich zwischen Elektrode und Schädel befindet, verhindert oder zumindest deutlich reduziert werden. Weiterhin wird das Signal-zu-Rausch-Verhältnis verbessert. Es ist denkbar, dass die erste umlaufende Dichtlippe in einem Stück aus dem Trägermaterial geformt sein kann.

Gemäß einer vorteilhaften Ausführungsform überragt eine erste zumindest teilweise umlaufende Dichtlippe zumindest teilweise eine, mehrere oder alle Primärelektroden und/oder zumindest teilweise eine, mehrere oder alle Sekundärelektroden um 0,05 mm bis 1 mm, insbesondere 0,1 mm bis 0,3 mm. Es hat sich gezeigt, dass diese Abmessungen die Flexibilität der Elektrodenanordnung gewährleisten und gleichzeitig eine effiziente Isolierung der Elektroden vor äußeren Einflüssen bereitstellen. Die Dichtlippe kann den Elektrodenkörper der jeweiligen Elektrode dabei in jeder Raumrichtung überragen. Beispielsweise kann die Dichtlippe den Elektrodenkörper in Normalenrichtung der Stimulationsoberfläche und/oder zentripetal zu der Stimulationsoberfläche überragen.

Jede in dieser Anmeldung beschriebene Isolierung, beispielsweise durch das Trägermaterial oder Dichtlippen, trägt auch zu einer verbesserten Signalqualität der Elektroden bei, sodass das Signal-zu-Rausch-Verhältnis verbessert wird.

Die Verbindungsstelle des elektrischen Leiters mit der Primärelektrode und/oder zumindest einer der Sekundärelektroden kann abgedichtet sein, insbesondere mit einem Epoxidharz oder einem anderen Dichtmittel. Dies hat den Vorteil, dass die empfindliche Verbindungsstelle vor äußeren Einflüssen wie zum Beispiel Flüssigkeiten geschützt wird, die zu einem Ausfall, zum Beispiel durch Korrosion, der jeweiligen Elektrode führen würde.

Vorteilhafterweise ist der elektrische Leiter aus einem Material gefertigt, welches, insofern es in elektrischem Kontakt mit dem Elektrodenmaterial steht, in wässriger Elektrolytlösung kein elektrochemisches Lokalelement bildet. Durch eine solche Materialauswahl wird die Wahrscheinlichkeit korrosiver Prozesse, die zu einer Beschädigung der Elektroden oder des elektrischen Leiters führen, verringert.

Die Aufgabe wird weiterhin durch ein Verfahren mit den Merkmalen des Anspruchs 13 gelöst.

Somit ist ein Verfahren zur Herstellung einer Elektrodenanordnung zur elektrischen Stimulation von Gewebe eines Lebewesens vorgesehen, insbesondere zur Herstellung einer Elektrodenanordnung gemäß den vorbeschriebenen Merkmalen.

In einem Verfahrensschritt a) werden, z.B. durch Laserschneiden oder Stanzen, Elektrodenkörper aus einer Folie eines Elektrodenmaterials mit geeigneter Dicke hergestellt. In einem Verfahrensschritt b) wird gegebenenfalls eine Oberflächenbeschichtung und/oder eine Strukturierung der Oberfläche auf zumindest einen Teil der Elektrodenfläche aufgebracht. In einem Verfahrensschritt c) wird an jedem Elektrodenkörper ein elektrischer Leiter angebracht oder der elektrische Leiter aus einem Stück zusammen mit den Elektroden hergestellt. In einem Verfahrensschritt d) wird gegebenenfalls ein Epoxidharz oder ein anderer elektrischer Isolator auf die Verbindungsstelle aufgebracht. In einem Verfahrensschritt e) wird eine Form der Elektrodenanordnung aus dem Trägermaterial hergestellt. In einem Verfahrensschritt f) wird der Elektrodenkörper sowie gegebenenfalls der elektrische Leiter und gegebenenfalls das Verstärkungselement eingebettet. In einem Verfahrensschritt g) wird ein elektrischer Leiter am Kontaktierungsabschnitt der Elektrodenanordnung angeschlossen.

Die Aufgabe wird weiterhin durch ein Verfahren mit den Merkmalen des Anspruchs 14 gelöst.

Somit ist ein Verfahren zur Herstellung einer Elektrodenanordnung zur elektrischen Stimulation von Gewebe eines Lebewesens vorgesehen, insbesondere zur Herstellung einer Elektrodenanordnung gemäß den vorbeschriebenen Merkmalen.

In einem Verfahrensschritt a) wird ein Trägermaterial bereitgestellt. In einem Verfahrensschritt b) wird ein oder werden mehrere Elektrodenkörper auf dem Trägermaterial durch einen Gasphasenabscheideprozess und/oder durch galvanische Abscheidung erzeugt. In einem Verfahrensschritt c) wird gegebenenfalls eine Oberflächenbeschichtung und/oder Strukturierung einer Oberfläche auf zumindest einen Teil der Elektrodenfläche aufgebracht. In einem Verfahrensschritt d) wird gegebenenfalls das Trägermaterial in ein weiteres isolierendes Trägermaterial eingebettet.

In einer vorteilhaften Ausführungsform der Verfahren wird wenigstens ein elektrischer Leiter zur elektrischen Verbindung des Elektrodenkörpers mit einem elektrischen Gerät oder einem anderen Elektrodenkörper durch einen Gasphasenabscheideprozess und/oder durch galvanische Abscheidung erzeugt und an den Elektrodenkörper angeschlossen.

Die Erfindung wird nachfolgend beispielhaft mit den beigefügten Zeichnungen näher erläutert. Es zeigen:
- Figur 1 -: einen schematischen Aufbau eines Elektrodenkörpers;
- Figur 2 -: eine schematische Darstellung eines Elektrodenkörpers mit einem angeschlossenen elektrischen Leiter und einer Isolierstoffhülse;
- Figur 3 -: eine schematische Darstellung des Elektrodenkörpers aus Figur 2 mit einem Trägermaterial;
- Figur 4 -: eine schematische Darstellung mehrerer Elektrodenkörper, die über elektrische Leiter an eine Energiequelle angeschlossen sind;
- Figur 5 -: eine schematische Darstellung eines in ein Trägermaterial eingebetteten Elektrodenkörpers in einer Seitenansicht;
- Figur 6 -: eine schematische Darstellung einer Elektrodenanordnung in einer Draufsicht;
- Figur 7 -: eine schematische Darstellung einer Elektrodenanordnung mit in ein Trägermaterial eingebetteten Elektroden in einer Seitenansicht;
- Figur 8 -: eine schematische Darstellung eines Verfahrens zur Herstellung einer Elektrodenanordnung;
- Figur 9 -: eine schematische Darstellung eines weiteren Verfahrens zur Herstellung einer Elektrodenanordnung.

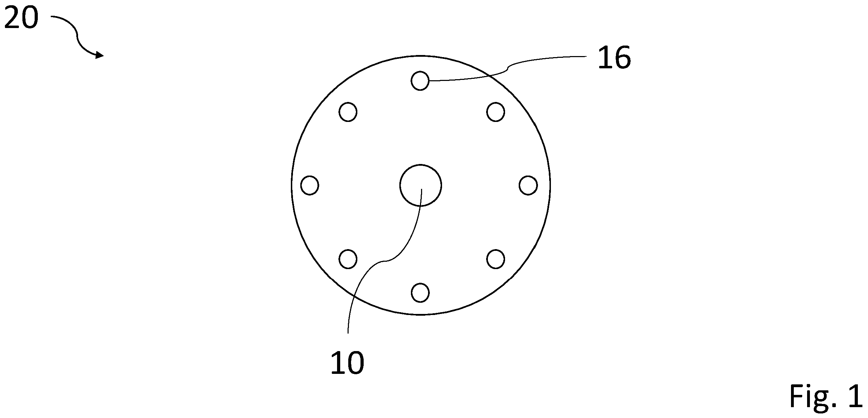

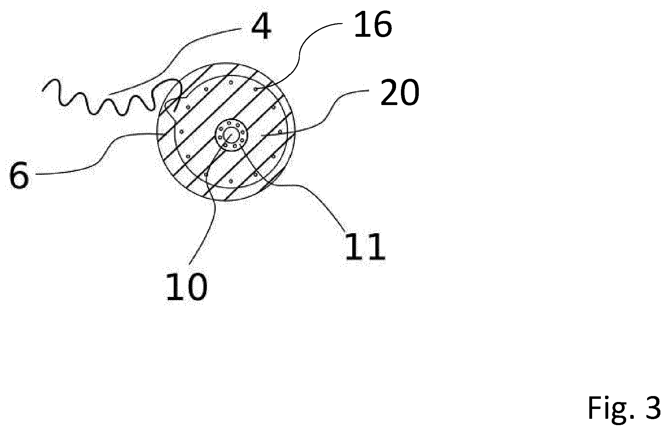

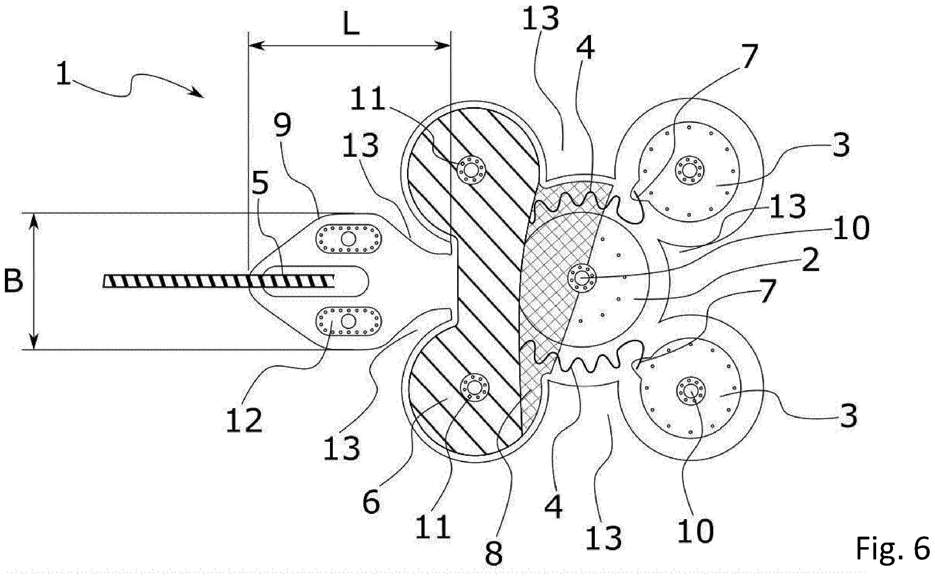

Figur 1 zeigt einen schematischen Aufbau eines Elektrodenkörpers 20. Der dargestellte Elektrodenkörper 20 ist dabei als Rundelektrode ausgeführt. Es sind aber auch andere Elektrodenformen denkbar. Deutlich wird, dass der Elektrodenkörper 20 eine Befestigungsöffnung 10 zur Befestigung an einem Lebewesen hat. Deutlich wird weiterhin, dass der Elektrodenkörper 20 über seinen Umfang verteilt angeordnete Ausnehmungen 16 hat. Durch die Ausnehmungen 16 wird eine verbesserte Fixierung des Elektrodenkörpers 20 in einem beispielsweise in Figur 3 gezeigten Trägermaterial 6 erreicht. Bei der Herstellung einer den Elektrodenkörper 20 aufnehmenden, beispielsweise in Figur 6 gezeigten Elektrodenanordnung 1 ist das Trägermaterial 6 zunächst in einem flüssigen Zustand, wobei das Trägermaterial 6 sich in den Ausnehmungen 16 verteilt und der Elektrodenkörper 20 damit nach dem Aushärten des Trägermaterials 6 fixiert gehalten werden kann.

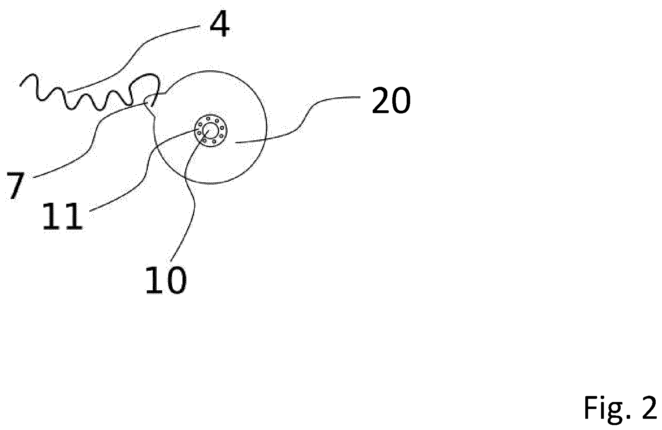

Figur 2 zeigt schematisch einen ebenfalls als Rundelektrode ausgeführten Elektrodenkörper 20 mit einem angeschlossenen elektrischen Leiter 4. Zum Anschluss des elektrischen Leiters 4 ist an dem Elektrodenkörper 20 ein Leiteranschluss 7 vorgesehen, der beispielsweise als aus der Kreisoberfläche der Rundelektrode herausragender Vorsprung ausgebildet sein kann, um die wirksame Stimulationsoberfläche des Elektrodenkörpers 20 nicht zu verringern. Der Leiteranschluss 7 ist vorliegend als tangentialer Leiteranschluss 7 ausgebildet, sodass der angeschlossene elektrische Leiter 4 den Elektrodenkörper 20 wie eine Tangente berührt. Hierdurch wird die Anschlussstelle bei einer auftretenden Zugkraft entlastet und somit eine Beschädigung des Leiteranschlusses 7 mit einem resultierenden Ausfall der Elektrode verhindert. Der elektrische Leiter 4 kann an den Leiteranschluss 7 des Elektrodenkörpers 20 angelötet oder angeschweißt sein, wobei es vorteilhaft ist, wenn die Verbindungsstelle gegen äußere Einflüsse zum Beispiel mit einem Epoxidharz abgedichtet ist. Zu erkennen ist, dass der elektrische Leiter 4 mäanderförmig verläuft. Dies verringert die Belastung des elektrischen Leiters 4, insbesondere in dessen Anschlussbereich am Leiteranschluss 7, die zum Beispiel durch Biegen und Ziehen einer den Elektrodenkörper aufnehmenden Elektrodenanordnung 1 entstehen können.

Weiterhin ist zu erkennen, dass der Elektrodenkörper 20 eine Befestigungsöffnung 10 aufweist, in die eine Isolierstoffhülse 11 eingesetzt ist. Dadurch kann der Elektrodenkörper 20 über ein Befestigungselement, beispielsweise eine Schraube, durch die Isolierstoffhülse 11 an einem Schädel des Lebewesens befestigt werden. Die Isolierstoffhülse 11 verhindert dabei, dass ein zu großes Drehmoment auf den Elektrodenkörper 20 übertragen wird und diesen damit beschädigt. Des Weiteren wird ein unerwünschter Stromfluss zwischen dem Elektrodenkörper 20 und dem Befestigungselement vermieden. Die Isolierstoffhülse 11 kann dabei zum Beispiel aus einem Polyetheretherketon bestehen. Durch die Befestigung des Elektrodenkörpers 20 über eine Befestigungsöffnung 10 kann eine exakte Lage des Elektrodenkörpers 20 gewährleistet werden, der ein erwünschtes elektrisches Feld in der Zielregion erzeugen kann.

In Figur 3 ist der Elektrodenkörper 20 aus Figur 2 dargestellt. In dieser Ansicht ist zu erkennen, dass der Elektrodenkörper 20 in ein schraffiert angedeutetes Trägermaterial 6 eingebettet ist. Das Trägermaterial 6 ist elektrisch isolierend und umgibt den Elektrodenkörper 20 vollständig bis auf die Stimulationsoberfläche. Das Trägermaterial 6 kann dabei zum Beispiel aus einem Silikon bestehen. Es sind aber auch andere Materialen denkbar, die die anwendungsbedingten Eigenschaften (Biokompatibilität, Flexibilität, elektrisch isolierend, etc.) erfüllen. Das Trägermaterial 6 ermöglicht eine einfache Anordnung und Fixierung des Elektrodenkörpers 20 in einer übergeordneten Struktur, insbesondere einer Elektrodenanordnung 1. Zudem wird durch das elektrisch isolierende Trägermaterial 6 das Auftreten von Leck- und Kurzschlussströmen verhindert oder zumindest reduziert.

Weiterhin weist der Elektrodenkörper 20 Ausnehmungen 16 auf. Das in einem Herstellungsprozess zunächst flüssige Trägermaterial 6 dringt in die Ausnehmungen 16 und unterstützt somit nach dem Aushärten die Fixierung des Elektrodenkörpers 20 in dem Trägermaterial 6.

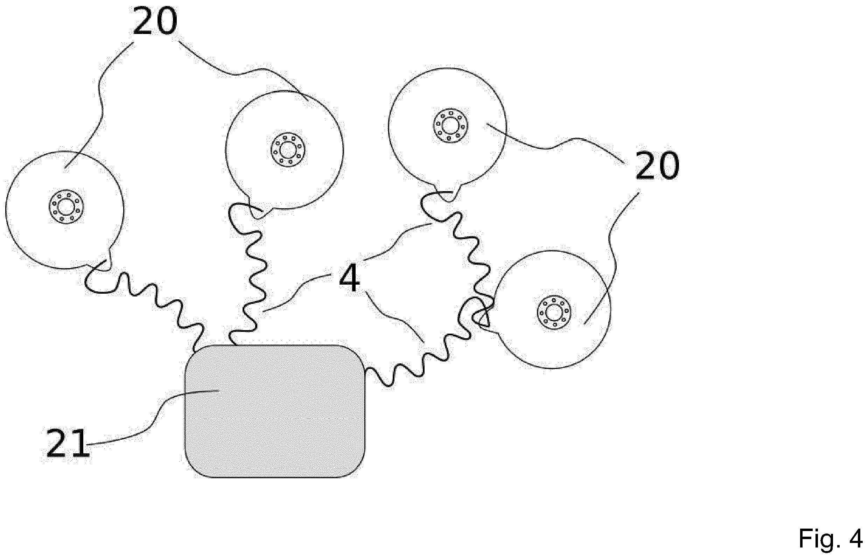

Figur 4 zeigt schematisch mehrere, nämlich insgesamt vier Elektrodenkörper 20, die über elektrische Leiter 4 mit einer Energiequelle 21 verbunden sind. Die Energiequelle 21 kann als Versorgungsquelle ausgebildet sind, um die Elektrodenkörper mit elektrischem Strom zu versorgen und hierdurch ihren Betrieb zu ermöglichen. Alternativ oder zusätzlich kann die Energiequelle 21 als Signalquelle ausgebildet sein, um gezielt elektrische Stimulationssignale an den Elektroden zu erzeugen.

Zu erkennen ist, dass die Elektrodenkörper 20 nicht unmittelbar mit der Energiequelle 21 verbunden sein müssen. Es ist genauso auch eine Anordnung denkbar, in der ein Elektrodenkörper 20 mit einem weiteren Elektrodenkörper 20 verbunden ist, der wiederum an die Energiequelle 21 angeschlossen ist. Hierzu verläuft der jeweilige elektrische Leiter 4 entweder zwischen einem Elektrodenkörper 20 und der Energiequelle 21 oder zwischen zwei Elektrodenkörpern 20. Durch eine mittelbare Verbindung eines Elektrodenkörpers 20 mit der Energiequelle 21 über einen weiteren Elektrodenkörper 20 können auch weiter von der Energiequelle 21 entfernte Geweberegionen erreicht werden, ohne längere elektrische Leiter 4 vorhalten zu müssen oder diese zu strecken und dadurch einem höheren Belastungsrisiko auszusetzen. Selbstverständlich können auch mehrere, also mehr als zwei Elektrodenkörper 20 mit einem weiteren Elektrodenkörper 20 verbunden werden. An dem zur Verbindung der elektrischen Leiter 4 vorgesehenen Leiteranschluss 7 können die mehreren Leiter 4 beispielsweise nebeneinander angelötet werden. Grundsätzlich ist es natürlich ebenfalls denkbar, einen längeren Leiter 4 für den räumlich entfernteren Elektrodenkörper 20 zu verwenden und einen weiteren, räumlich näheren Elektrodenkörper 20 als Abzweig elektrisch anzubinden.

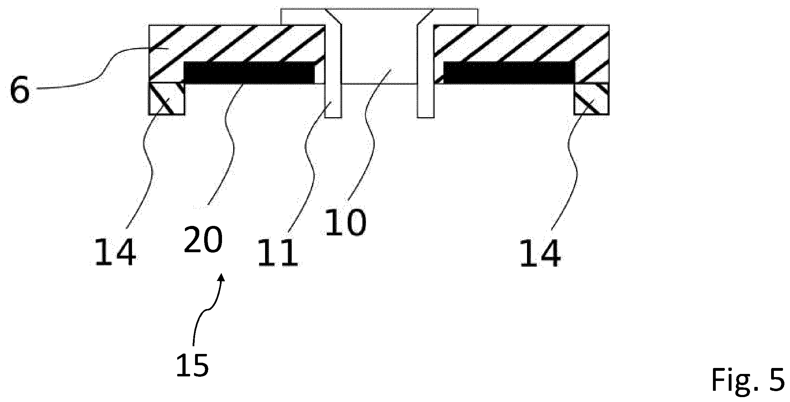

Figur 5 zeigt den in ein Trägermaterial 6 eingebetteten Elektrodenkörper 20 in einer Seitenansicht. Zu erkennen ist, dass der Elektrodenkörper 20 eine durchgehende Befestigungsöffnung 10 hat, wobei in der Befestigungsöffnung 10 eine Isolierstoffhülse 11 angeordnet ist und wobei der Elektrodenkörper 20 mittels eines nicht gezeigten Befestigungselements über die Isolierstoffhülse 11 an einer Knochen- oder anderweitigen Gewebestruktur des Lebewesens befestigt werden kann. Es wird deutlich, dass das Trägermaterial 6 eine erste umlaufende Dichtlippe 14 an einer Kontaktierungsseite 15 des Elektrodenkörpers 20 hat. Die umlaufende Dichtlippe 14 kann dabei einstückig aus demselben Material des Trägermaterials 6 gebildet sein. Auf diese Weise kann der Elektrodenkörper 20 trotz Unebenheiten an der zu implantierenden Stelle befestigt werden, ohne dass es zu Deformationen an dem Elektrodenkörper 20 kommt. Dabei ist es auch denkbar, dass die umlaufende Dichtlippe 14 den Elektrodenkörper 20 teilweise überragt, wodurch eine verbesserte Fixierung des Elektrodenkörpers 20 in dem Trägermaterial 6 ermöglicht wird. Weiterhin erkennbar ist, dass der Elektrodenkörper 20 auf der Kontaktierungsseite 15 freiliegend und auf der anderen Seite vom Trägermaterial 6 isolierend überdeckt ist. Der Elektrodenkörper 20 ist damit im Trägermaterial 6 eingebettet. Dies ermöglicht eine effiziente Isolierung des Elektrodenkörpers 20 nach außen und gewährleistet zugleich die Generierung eines erwünschten elektrischen Feldes in einer Körperregion zur Kontaktierungsseite 15 hin. Es ist auch denkbar, dass die Isolierstoffhülse 11 eine zweite, nicht gezeigte umlaufende Dichtlippe hat, die diesen Effekt noch zusätzlich begünstigt.

Figur 6 zeigt in einer schematischen Darstellung eine Elektrodenanordnung 1 in einer Draufsicht. Zur Verdeutlichung ist die Elektrodenanordnung 1 teilweise freigeschnitten, wobei unterschiedliche Schichten der Elektrodenanordnung 1 zu erkennen sind. Die Elektrodenanordnung 1 hat eine Primärelektrode 2, wobei die Primärelektrode 2 von vier Sekundärelektroden 3 umgeben ist. Die Sekundärelektroden 3 sind dabei auf einer gemeinsamen Kreisbahn um die Primärelektrode 2 angeordnet. Die Primärelektrode 2 und die Sekundärelektroden 3 sind dabei über jeweils einen elektrischen Leiter 4 mit einem Anschlusselement 5 verbunden, wobei das Anschlusselement 5 zum Anschluss an eine Energiequelle 21 eingerichtet ist. Die Primär- und Sekundärelektroden 2, 3 können vorteilhafterweise weitere Merkmale des vorbeschriebenen Elektrodenkörpers 20 aufweisen.

Es wird deutlich, dass die Komponenten (Primärelektrode 2, Sekundärelektroden 3, elektrische Leiter 4 und Anschlusselement 5) an einem Trägermaterial 6 angeordnet sind. Das Trägermaterial 6 kann dabei zum Beispiel aus einem Silikon bestehen. Es sind aber auch andere Materialen denkbar, die die anwendungsbedingten Eigenschaften (Biokompatibilität, Flexibilität, elektrisch isolierend, etc.) erfüllen. Es wird deutlich, dass die Primärelektrode 2 und die Sekundärelektroden 3 sowie die elektrischen Leiter 4 in das Trägermaterial 6 eingebettet sind. Weiterhin wird deutlich, dass die elektrischen Leiter 4 der hinteren, vom Anschlusselement 5 weiter entfernten Sekundärelektroden 3 mäanderförmig um die Primärelektrode 1 angeordnet sind, wobei die mäanderförmige Anordnung auf einer gebogenen Bahn um die Primärelektrode 2 verläuft. Auf diese Weise kann die Belastung auf die elektrischen Leiter 4 und damit auch die Ausfallwahrscheinlichkeit der Sekundärelektroden 3 verringert werden. Die elektrischen Leiter 4 sind über eine Verbindungsstelle mit den Sekundärelektroden 3 verbunden, wobei die Verbindungsstelle ein tangentialer Leiteranschluss 7 ist. Der elektrische Leiter 4 kann dabei an die Sekundärelektrode 3 angelötet oder angeschweißt sein, wobei es vorteilhaft ist, wenn die Verbindungsstelle gegen äußere Einflüsse zum Beispiel mit einem Epoxidharz abgedichtet ist.

An der Elektrodenanordnung 1 ist ein Verstärkungselement 8 in Form eines chirurgischen Netzes angeordnet, wobei das Verstärkungselement 8 zwischen den äußeren Schichten des Trägermaterials eingezogen ist. Das Verstärkungselement 8 ist dabei zwischen der Primärelektrode 2 und den Sekundärelektroden 3 zur Verbesserung der Reißfestigkeit angeordnet.

Es ist erkennbar, dass das Anschlusselement 5 an einem Kontaktierungsabschnitt 9 angeordnet ist, wobei der Kontaktierungsabschnitt 9 seitlich von der Elektrodenanordnung 1 abragt und eine größere Länge L als Breite B hat. Die längliche Ausgestaltung des Kontaktierungsabschnittes 9 hat den Vorteil, dass das Anschlusselement 5 von der Primärelektrode 2 und den Sekundärelektroden 3 entkoppelt ist, wobei auf den Kontaktierungsabschnitt 9 einwirkende Kräfte über die Länge L des Kontaktierungsabschnittes 9 soweit kompensiert werden, dass diese Kräfte gar nicht oder schwach an die Elektrodenanordnung 1 weitergeleitet werden. Um die Flexibilität des Kontaktierungsabschnittes 9 zu gewährleisten, kann es von Vorteil sein, wenn das Verstärkungselement 8 nicht am Kontaktierungsabschnitt 9 angeordnet ist und sich lediglich bis zum Übergansgereich zum Kontaktierungsabschnitt 9 hin erstreckt oder sich ähnlich eines Knickschutzes graduell verkleinernd in den Kontaktierungsabschnitt 9 hinein erstreckt.

Die Elektrodenanordnung 1 ist dazu eingerichtet, zum Beispiel zwischen Kopfhaut und Schädel eines Lebewesens implantiert zu werden. Dabei haben die Primärelektrode 2 und die Sekundärelektroden 3 Befestigungsöffnungen 10, wobei in jeweils eine Befestigungsöffnung 10 eine Isolierstoffhülse 11 eingesetzt ist. Die Elektrodenanordnung 1 kann dann über Befestigungselemente wie zum Beispiel über Schrauben durch die Isolierstoffhülse 11 an dem Schädel des Lebewesens befestigt werden. Die Isolierstoffhülse 11 verhindert dabei, dass ein zu großes Drehmoment auf die Primärelektrode 2 oder die Sekundärelektrode 3 übertragen wird und diese damit beschädigt. Des Weiteren wird ein unerwünschter Stromfluss zwischen den Elektroden (Primärelektrode 2 und Sekundärelektroden 3) und dem Befestigungselement vermieden. Die Isolierstoffhülse 11 kann dabei zum Beispiel aus einem Polyetheretherketon bestehen. Durch die Befestigung der Elektrodenanordnung 1 über Befestigungsöffnungen 10 an den jeweiligen Elektroden (Primärelektrode 2 und Sekundärelektroden 3) kann eine exakte Lage der Elektroden gewährleistet werden, die ein erwünschtes elektrisches Feld in der Zielregion erzeugen können. Weiterhin ist erkennbar, dass an dem Kontaktierungsabschnitt 9 zwei Befestigungselemente 12 zur Schraubbefestigung an einem Lebewesen angeordnet sind. Der Kontaktierungsabschnitt 9 kann durch die Befestigungselemente 12 an dem Schädel des Lebewesens befestigt werden.

Deutlich wird, dass die Elektrodenanordnung 1 jeweils zwischen den Sekundärelektroden 3 eine Aussparung 13 hat. Die Aussparungen 13 erhöhen die Flexibilität der Elektrodenanordnung 1, insbesondere im Bereich der Sekundärelektroden 3. Da der Knochen eines Lebewesens in der Regel nicht eben ausgebildet ist, die Elektroden (Primärelektrode 2 und Sekundärelektroden 3) aber exakt am Knochen positioniert werden müssen, ist es vorteilhaft, wenn die Flexibilität der Elektrodenanordnung 1 entsprechend hoch ist, damit die Elektroden 2, 3 in entsprechender Nähe zum Knochen befestigt werden können und die Elektrodenanordnung 1 sich gut der Knochenform anpassen kann.

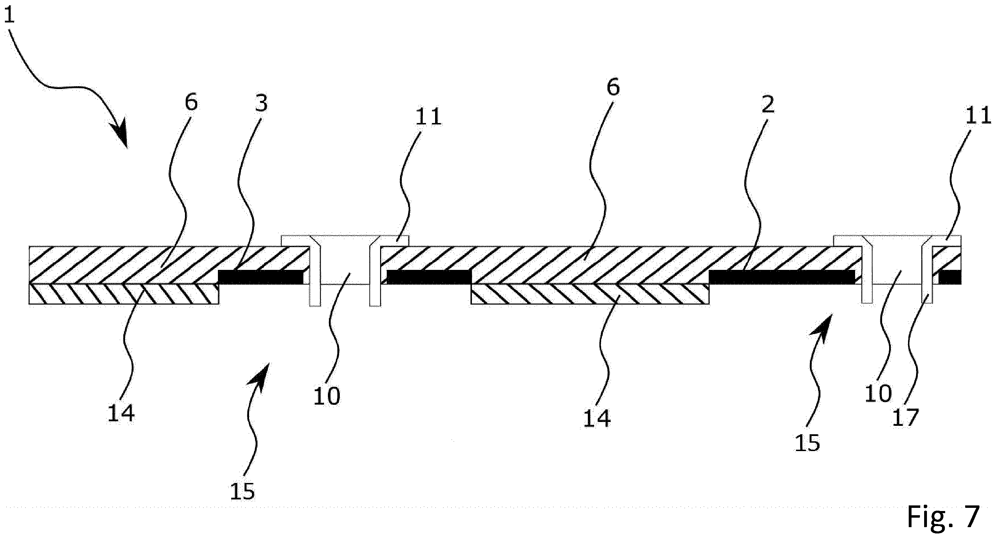

Figur 7 zeigt eine schematische Darstellung einer Primärelektrode 2 und einer Sekundärelektrode 3 in dem Trägermaterial 6 in einer Seitenansicht. Zu erkennen ist, dass die Primärelektrode 2 und die Sekundärelektrode 3 jeweils eine durchgehende Befestigungsöffnung 10 haben, wobei in der Befestigungsöffnung 10 eine Isolierstoffhülse 11 angeordnet ist und wobei die Elektrodenanordnung 1 mittels Befestigungselementen über die Isolierstoffhülse 11 an einer Knochen- oder Gewebestruktur eines Lebewesens befestigt werden kann.

Es wird deutlich, dass das Trägermaterial 6 eine erste umlaufende Dichtlippe 14 an der freiliegenden Kontaktierungsseite 15 der Sekundärelektroden 3 hat. Die umlaufende Dichtlippe 14 kann dabei einstückig aus demselben Material des Trägermaterials 6 gebildet sein. Auf diese Weise kann die Elektrodenanordnung 1 trotz Unebenheiten an der zu implantierenden Stelle befestigt werden, ohne dass es zu Deformationen an den Sekundärelektroden 3 kommt. Dabei ist es auch denkbar, dass die umlaufende Dichtlippe 14 die Sekundärelektrode 3 teilweise überragt, sodass eine verbesserte Fixierung der Sekundärelektrode 3 in dem Trägermaterial 6 ermöglicht wird. Weiterhin erkennbar ist, dass die Sekundärelektroden 3 auf der Kontaktierungsseite 15 freiliegend und auf der anderen Seite vom Trägermaterial 6 isolierend überdeckt sind. Die Sekundärelektroden 3 sind damit im Trägermaterial 6 eingebettet. Dies ermöglicht eine effiziente Isolierung der Sekundärelektroden 3 nach außen und gewährleistet zugleich die Generierung eines erwünschten elektrischen Feldes in einer Körperregion zur Kontaktierungsseite 15 hin. Es ist auch denkbar, dass die Isolierstoffhülse 11 eine zweite umlaufende Dichtlippe 17 hat, die diesen Effekt noch zusätzlich begünstigt. Es ist vorteilhaft, wenn alle Elektroden der Elektrodenanordnung 1 diese Ausgestaltungen aufweisen.

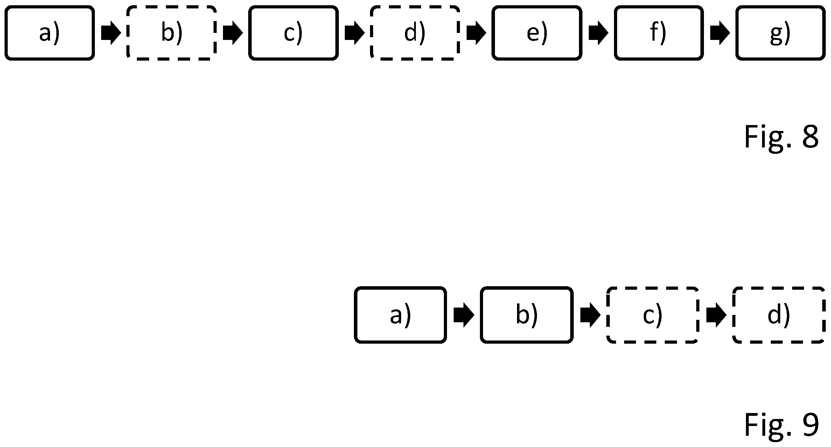

Figur 8 zeigt schematisch ein Verfahren zur Herstellung einer Elektrodenanordnung zur elektrischen Stimulation von Gewebe eines Lebewesens, insbesondere zur Herstellung einer Elektrodenanordnung 1 gemäß den vorbeschriebenen Merkmalen.

In einem Verfahrensschritt a) werden Elektrodenkörper 20 aus einer Folie eines Elektrodenmaterials mit geeigneter Dicke hergestellt, z.B. durch Ausstanzen oder durch Ausschneiden, z.B. mit einem Laser. In einem optionalen Verfahrensschritt b) wird eine Oberflächenbeschichtung und/oder eine Strukturierung der Oberfläche auf zumindest einen Teil der Elektrodenfläche aufgebracht. In einem Verfahrensschritt c) wird an jedem Elektrodenkörper 20 ein elektrischer Leiter 4 angebracht. In einem optionalen Verfahrensschritt d) wird ein Epoxidharz oder ein anderer elektrischer Isolator auf die Verbindungsstelle aufgebracht. In einem Verfahrensschritt e) wird eine Form der Elektrodenanordnung 1 aus dem Trägermaterial 6 gegossen. In einem Verfahrensschritt f) wird der Elektrodenkörper 20 sowie optional der elektrische Leiter 40 und optional das Verstärkungselement 8 eingebettet. In einem Verfahrensschritt g) wird ein elektrischer Leiter 4 am Kontaktierungsabschnitt 9 der Elektrodenanordnung 1 angeschlossen.

Figur 9 zeigt schematisch ein weiteres Verfahren zur Herstellung einer Elektrodenanordnung 1 zur elektrischen Stimulation von Gewebe eines Lebewesens, insbesondere zur Herstellung einer Elektrodenanordnung 1 gemäß den vorbeschriebenen Merkmalen. In einem Verfahrensschritt a) wird ein Trägermaterial 6 bereitgestellt. In einem Verfahrensschritt b) werden ein oder werden mehrere Elektrodenkörper 20 auf dem Trägermaterial 6 durch einen Gasphasenabscheideprozess und/oder durch galvanische Abscheidung erzeugt. In einem optionalen Verfahrensschritt c) wird eine Oberflächenbeschichtung und/oder Strukturierung einer Oberfläche auf zumindest einen Teil der Elektrodenfläche aufgebracht. In einem optionalen Verfahrensschritt d) wird das Trägermaterial 6 in ein weiteres isolierendes Trägermaterial eingebettet.

Die Figuren verstehen sich als ein mögliches Ausführungsbeispiel. Andere Formen der erfindungsgemäßen Lehre sind weiterhin denkbar. Des Weiteren sind die Ausgestaltungen des Ausführungsbeispiels nicht untrennbar miteinander verknüpft, sodass z.B. die Ausführung der Erfindung nicht abhängig von den speziell beschriebenen Ausgestaltungen des Ausführungsbeispiels ist. So ist eine Variabilität zum Beispiel von der Anzahl oder Lagerung der einzelnen Elemente wie zum Beispiel der Primärelektrode 2, Sekundärelektroden 3, Aussparungen 13 und/oder Ausnehmungen 16 jederzeit denkbar.

## Patentansprüche

1. Elektrodenanordnung (1) zur elektrischen Stimulation von Gewebe eines Lebewesens, insbesondere Elektrodenanordnung (1) für die Neurostimulation, mit wenigstens einer Primärelektrode (2) und wenigstens einer Sekundärelektrode (3), wobei eine, mehrere oder alle Primärelektroden (2) durch jeweils einen Elektrodenkörper (20) gebildet sind und/oder eine, mehrere oder alle Sekundärelektroden (3) jeweils durch einen Elektrodenkörper (20) gebildet sind, wobei die Primärelektrode (2) und die Sekundärelektrode (3) an einem gemeinsamen Trägermaterial (6) angeordnet sind, **dadurch gekennzeichnet, dass** das Trägermaterial (6) eine erste zumindest teilweise umlaufende Dichtlippe (14) an einer freiliegenden Kontaktierungsseite (15) des Elektrodenkörpers (20) hat, die zumindest teilweise eine, mehrere oder alle Primärelektroden (2) und/oder zumindest teilweise eine, mehrere oder alle Sekundärelektroden um 0,05 mm bis 1 mm, insbesondere 0,1 mm bis 0,3 mm, überragt..

2. Elektrodenanordnung (1) nach Anspruch 1, **dadurch gekennzeichnet, dass** die Elektrodenanordnung (1) dazu eingerichtet ist, an einer Stelle zwischen dem Schädel und der Kopfschwarte eines Lebewesens angeordnet zu werden, und wobei die Elektrodenanordnung (1) eine Stimulationsoberfläche aufweist, die dazu eingerichtet ist, in Kontakt mit dem Gewebe des Lebewesens gebracht zu werden, um eine elektrische Stimulation des Gewebes durch Wechselstrom- und/oder Gleichstrom-Pulse zu erzeugen, wobei die Elektrodenanordnung (1) eines oder mehrere der nachfolgenden Merkmale a) bis d) aufweist:
a) eine Gesamtgröße der Stimulationsoberfläche der Primärelektroden (2) und/oder eine Gesamtgröße der Stimulationsoberfläche aller Sekundärelektroden (3) mit einem Flächeninhalt von wenigstens 50 mm²,
b) eine Gesamtgröße der Stimulationsoberfläche der Primärelektroden (2) und/oder eine Gesamtgröße der Stimulationsoberfläche aller Sekundärelektroden (3) mit einem Flächeninhalt von wenigstens 20 mm² und zusätzlich eine die wirksame Stimulationsoberfläche vergrößernde Oberflächenbehandlung,
c) eine Fixierungsstruktur für die Fixierung der Elektrodenanordnung (1) am Gewebe eines Lebewesens,
d) die Elektrodenanordnung (1) ist bis auf die Stimulationsoberflächen der Primär- und Sekundärelektroden von einem elektrisch isolierenden Trägermaterial (6) vollständig umgeben.

3. Elektrodenanordnung (1) nach Anspruch 1 oder 2, **dadurch gekennzeichnet, dass** an dem Trägermaterial (6) ein Verstärkungselement (8), insbesondere ein Verstärkungsnetz, angeordnet ist.

4. Elektrodenanordnung (1) nach einem der Ansprüche 1 bis 3, **dadurch gekennzeichnet, dass** der zumindest eine elektrische Leiter (4) zumindest teilweise vom Trägermaterial (6) umgeben ist.

5. Elektrodenanordnung (1) nach einem der Ansprüche 1 bis 4, **dadurch gekennzeichnet, dass** die Dicke der Elektrodenanordnung (1) kleiner als 3 mm, insbesondere kleiner als 2 mm oder als 1 mm, ist.

6. Elektrodenanordnung (1) nach einem der Ansprüche 1 bis 5, **dadurch gekennzeichnet, dass** die Elektrodenanordnung (1) wenigstens eine Primärelektrode (2) und wenigstens zwei Sekundärelektroden (3) aufweist.

7. Elektrodenanordnung (1) nach einem der Ansprüche 1 bis 6, **dadurch gekennzeichnet, dass** wenigstens einer der elektrischen Leiter (4) der Sekundärelektroden (3) mäanderförmig um die Primärelektrode (2) angeordnet ist, wobei die mäanderförmige Anordnung des elektrischen Leiters (4) entlang einer gebogenen Bahn um die Primärelektrode (2) verläuft.

8. Elektrodenanordnung (1) nach einem der Ansprüche 1 bis 7, **dadurch gekennzeichnet, dass** die Elektrodenanordnung (1) einen Kontaktierungsabschnitt (9) hat, wobei das Anschlusselement (5) an dem Kontaktierungsabschnitt (9) angeordnet ist und wobei der Kontaktierungsabschnitt (9) seitlich von der Elektrodenanordnung (1) abragt.

9. Elektrodenanordnung (1) nach Anspruch 8, **dadurch gekennzeichnet, dass** der Kontaktierungsabschnitt (9) eine größere Länge als Breite hat.

10. Elektrodenanordnung (1) nach einem der Ansprüche 1 bis 9, **dadurch gekennzeichnet, dass** die Sekundärelektroden (3) auf wenigstens einer Kreisbahn um die Primärelektrode (2) angeordnet sind.

11. Elektrodenanordnung (1) nach einem der Ansprüche 1 bis 10, **dadurch gekennzeichnet, dass** der Rand wenigstens einer der Sekundärelektroden (3) in einem Abstand von 1 mm bis 8 mm, insbesondere in einem Abstand von 2 mm bis 6 mm, zum Rand der Primärelektrode (2) angeordnet ist.

12. Elektrodenanordnung (1) nach einem der Ansprüche 1 bis 11, **dadurch gekennzeichnet, dass** eine erste zumindest teilweise umlaufende Dichtlippe (14) zumindest teilweise eine, mehrere oder alle Primärelektroden (2) und/oder zumindest teilweise eine, mehrere oder alle Sekundärelektroden um 0,05 mm bis 1 mm, insbesondere 0,1 mm bis 0,3 mm, überragt.

13. Verfahren zur Herstellung einer Elektrodenanordnung (1) zur elektrischen Stimulation von Gewebe eines Lebewesens nach einem der vorhergehenden Ansprüche, **gekennzeichnet durch** folgende Schritte:
a) Herstellen von Elektrodenkörpern (20), insbesondere durch Laserschneiden, aus einer Folie eines Elektrodenmaterials mit geeigneter Dicke,
b) Ggf. Aufbringen einer Oberflächenbeschichtung und/oder Strukturierung der Oberfläche auf zumindest einen Teil der Elektrodenfläche,
c) Anbringen oder Herstellen je eines elektrischen Leiters (4) an jedem Elektrodenkörper (20),
d) Ggf. Aufbringen eines Epoxidharzes auf die Verbindungsstelle,
e) Gießen einer Form der Elektrodenanordnung (1) aus dem Trägermaterial (6),
f) Einbetten der Elektrodenkörper (20) und ggf. der elektrischen Leiter (4) und ggf. des Verstärkungselements (8),
g) Anschließen eines elektrischen Leiters (4) am Kontaktierungsabschnitt (9) der Elektrodenanordnung (1).

14. Verfahren zur Herstellung einer Elektrodenanordnung (1) zur elektrischen Stimulation von Gewebe eines Lebewesens nach einem der vorhergehenden Ansprüche, **gekennzeichnet durch** folgende Schritte:
a) Bereitstellen eines Trägermaterials (6),
b) Erzeugen eines oder mehrerer Elektrodenkörper (20) auf dem Trägermaterial (6) durch einen Gasphasenabscheideprozess und/oder durch galvanische Abscheidung,
c) Ggf. Aufbringen einer Oberflächenbeschichtung und/oder Strukturierung der Oberfläche auf zumindest einen Teil der Elektrodenfläche,
d) Ggf. Einbetten des Trägermaterials (6) in ein weiteres isolierendes Trägermaterial.

## Claims

1. Electrode arrangement (1) for the electrical stimulation of tissue of a living being, in particular electrode arrangement (1) for neurostimulation, having at least one primary electrode (2) and at least one secondary electrode (3), one, several or all primary electrodes (2) each being formed by an electrode body (20) and/or one, several or all secondary electrodes (3) each being formed by an electrode body (20), wherein the primary electrode (2) and the secondary electrode (3) are arranged on a common carrier material (6), **characterized in that** the carrier material (6) has a first at least partially circumferential sealing lip (14) on an exposed contacting side (15) of the electrode body (20), which at least partially projects beyond one, several or all primary electrodes (2) and/or at least partially beyond one, several or all secondary electrodes by 0.05 mm to 1 mm, in particular 0.1 mm to 0.3 mm.

2. Electrode arrangement (1) according to claim 1, **characterized in that** the electrode arrangement (1) is adapted to be placed at a location between the skull and the scalp of a living being, and wherein the electrode arrangement (1) comprises a stimulation surface adapted to be brought into contact with the tissue of the living being in order to generate electrical stimulation of the tissue by alternating current and/or direct current pulses, wherein the electrode arrangement (1) comprises one or more of the following features a) to d):
a) a total size of the stimulation surface of the primary electrodes (2) and/or a total size of the stimulation surface of all secondary electrodes (3) with an area of at least 50 mm²,
b) a total size of the stimulation surface of the primary electrodes (2) and/or a total size of the stimulation surface of all secondary electrodes (3) with a surface area of at least 20 mm² and additionally a surface treatment increasing the effective stimulation surface,
c) a fixing structure for fixing the electrode arrangement (1) to the tissue of a living being,
d) the electrode arrangement (1) is completely surrounded by an electrically insulating carrier material (6) except for the stimulation surfaces of the primary and secondary electrodes.

3. Electrode arrangement (1) according to claim 1 or 2, **characterized in that** a reinforcing element (8), in particular a reinforcing mesh, is arranged on the carrier material (6).

4. Electrode arrangement (1) according to one of claims 1 to 3, **characterized in that** the at least one electrical conductor (4) is at least partially surrounded by the carrier material (6).

5. Electrode arrangement (1) according to one of claims 1 to 4, **characterized in that** the thickness of the electrode arrangement (1) is less than 3 mm, in particular less than 2 mm or less than 1 mm.

6. Electrode arrangement (1) according to one of claims 1 to 5, **characterized in that** the electrode arrangement (1) has at least one primary electrode (2) and at least two secondary electrodes (3).

7. Electrode arrangement (1) according to one of claims 1 to 6, **characterized in that** at least one of the electrical conductors (4) of the secondary electrodes (3) is arranged in a meandering manner around the primary electrode (2), the meandering arrangement of the electrical conductor (4) running along a curved path around the primary electrode (2).

8. Electrode arrangement (1) according to one of claims 1 to 7, **characterized in that** the electrode arrangement (1) has a contacting section (9), wherein the connecting element (5) is arranged on the contacting section (9) and wherein the contacting section (9) projects laterally from the electrode arrangement (1).

9. Electrode arrangement (1) according to claim 8, **characterized in that** the contacting section (9) has a greater length than width.

10. Electrode arrangement (1) according to one of claims 1 to 9, **characterized in that** the secondary electrodes (3) are arranged on at least one circular path around the primary electrode (2).

11. Electrode arrangement (1) according to one of claims 1 to 10, **characterized in that** the edge of at least one of the secondary electrodes (3) is arranged at a distance of 1 mm to 8 mm, in particular at a distance of 2 mm to 6 mm, from the edge of the primary electrode (2).

12. Electrode arrangement (1) according to one of claims 1 to 11, **characterized in that** a first at least partially circumferential sealing lip (14) at least partially projects beyond one, several or all primary electrodes (2) and/or at least partially beyond one, several or all secondary electrodes by 0.05 mm to 1 mm, in particular 0.1 mm to 0.3 mm.

13. Method for producing an electrode arrangement (1) for the electrical stimulation of tissue of a living being according to one of the preceding claims, **characterized by** the following steps:
a) Manufacture of electrode bodies (20), in particular by laser cutting, from a sheet of an electrode material of suitable thickness,
b) If necessary, application of a surface coating and/or structuring of the surface on at least part of the electrode surface,
c) Attachment or manufacture of one electrical conductor (4) to each electrode body (20),
d) If necessary, apply an epoxy resin to the joint,
e) Casting a mold of the electrode assembly (1) from the carrier material (6),
f) Embedding the electrode bodies (20) and, if applicable, the electrical conductors (4) and, if applicable, the reinforcing element (8),
g) Connecting an electrical conductor (4) to the contacting section (9) of the electrode arrangement (1).

14. Method for producing an electrode arrangement (1) for electrical stimulation of tissue of a living being according to one of the preceding claims, **characterized by** the following steps:
a) Providing a carrier material (6),
b) Producing one or more electrode bodies (20) on the carrier material (6) by a vapor deposition process and/or by electrodeposition,
c) If necessary, application of a surface coating and/or structuring of the surface on at least part of the electrode surface,
d) If necessary, embed the carrier material (6) in another insulating carrier material.

## Revendications

1. Ensemble d'électrodes (1) pour la stimulation électrique du tissu d'un être vivant, en particulier ensemble d'électrodes (1) pour la neurostimulation, comprenant au moins une électrode primaire (2) et au moins une électrode secondaire (3), une, plusieurs ou toutes les électrodes primaires (2) étant formées chacune par un corps d'électrode (20), et/ou une, plusieurs ou toutes les électrodes secondaires (3) étant formées chacune par un corps d'électrode (20), l'électrode primaire (2) et l'électrode secondaire (3) étant disposées sur un matériau de support (6) commun,
**caractérisé en ce que** le matériau de support (6) présente une première lèvre d'étanchéité (14) au moins partiellement périphérique sur un côté de mise en contact dégagé (15) du corps d'électrode (20), qui dépasse au moins partiellement une, plusieurs ou toutes les électrodes primaires (2) et/ou au moins partiellement une, plusieurs ou toutes les électrodes secondaires d'une valeur de 0,05 mm à 1 mm, en particulier de 0,1 mm à 0,3 mm.

2. Ensemble d'électrodes (1) selon la revendication 1,
**caractérisé en ce que** l'ensemble d'électrodes (1) est conçu pour être placé à un endroit entre le crâne et le cuir chevelu d'un être vivant, l'ensemble d'électrodes (1) présentant une surface de stimulation conçue pour être mise en contact avec le tissu de l'être vivant afin de générer une stimulation électrique du tissu par des impulsions de courant alternatif et/ou de courant continu, l'ensemble d'électrodes (1) présentant un ou plusieurs des éléments suivants a) à d) :
a) une taille totale de la surface de stimulation des électrodes primaires (2) et/ou une taille totale de la surface de stimulation de toutes les électrodes secondaires (3), qui a une superficie d'au moins 50 mm²,
b) une taille totale de la surface de stimulation des électrodes primaires (2) et/ou une taille totale de la surface de stimulation de toutes les électrodes secondaires (3), qui a une superficie d'au moins 20 mm² et, en supplément, un traitement de surface qui augmente la surface de stimulation efficace,
c) une structure de fixation pour fixer l'ensemble d'électrodes (1) au tissu d'un être vivant,
d) l'ensemble d'électrodes (1) est entièrement entouré d'un matériau de support électriquement isolant (6), à l'exception des surfaces de stimulation des électrodes primaires et secondaires.

3. Ensemble d'électrodes (1) selon la revendication 1 ou 2,
**caractérisé en ce qu'**un élément de renforcement (8), en particulier un filet de renforcement, est disposé sur le matériau de support (6).

4. Ensemble d'électrodes (1) selon l'une des revendications 1 à 3,
**caractérisé en ce que** ledit au moins un conducteur électrique (4) est au moins partiellement entouré du matériau de support (6).

5. Ensemble d'électrodes (1) selon l'une des revendications 1 à 4,
**caractérisé en ce que** l'épaisseur de l'ensemble d'électrodes (1) est inférieure à 3 mm, en particulier inférieure à 2 mm ou à 1 mm.

6. Ensemble d'électrodes (1) selon l'une des revendications 1 à 5,
**caractérisé en ce que** l'ensemble d'électrodes (1) comprend au moins une électrode primaire (2) et au moins deux électrodes secondaires (3).

7. Ensemble d'électrodes (1) selon l'une des revendications 1 à 6,
**caractérisé en ce que** l'un au moins des conducteurs électriques (4) des électrodes secondaires (3) est disposé en méandres autour de l'électrode primaire (2), la disposition en méandres du conducteur électrique (4) s'étendant le long d'une trajectoire incurvée autour de l'électrode primaire (2).

8. Ensemble d'électrodes (1) selon l'une des revendications 1 à 7,
**caractérisé en ce que** l'ensemble d'électrodes (1) comprend une partie de mise en contact (9), l'élément de connexion (5) étant disposé sur la partie de mise en contact (9), et la partie de mise en contact (9) faisant saillie latéralement de l'ensemble d'électrodes (1).

9. Ensemble d'électrodes (1) selon la revendication 8,
**caractérisé en ce que** la partie de mise en contact (9) a une longueur supérieure à sa largeur.

10. Ensemble d'électrodes (1) selon l'une des revendications 1 à 9,
**caractérisé en ce que** les électrodes secondaires (3) sont disposées sur au moins une trajectoire circulaire autour de l'électrode primaire (2).

11. Ensemble d'électrodes (1) selon l'une des revendications 1 à 10,
**caractérisé en ce que** le bord de l'une au moins des électrodes secondaires (3) est disposé à une distance de 1 mm à 8 mm, en particulier à une distance de 2 mm à 6 mm, du bord de l'électrode primaire (2).

12. Ensemble d'électrodes (1) selon l'une des revendications 1 à 11,
**caractérisé en ce qu'**une première lèvre d'étanchéité (14) au moins partiellement périphérique dépasse au moins partiellement une, plusieurs ou toutes les électrodes primaires (2) et/ou au moins partiellement une, plusieurs ou toutes les électrodes secondaires d'une valeur de 0,05 mm à 1 mm, en particulier de 0,1 mm à 0,3 mm.

13. Procédé de fabrication d'un ensemble d'électrodes (1) pour la stimulation électrique du tissu d'un être vivant selon l'une des revendications précédentes,
**caractérisé par** les étapes suivantes consistant à :
a) réaliser des corps d'électrode (20), en particulier par découpe au laser, à partir d'une feuille d'un matériau d'électrode ayant une épaisseur appropriée,
b) le cas échéant, appliquer un revêtement de surface et/ou une structuration de surface sur au moins une partie de la surface de l'électrode,
c) monter ou réaliser un conducteur électrique (4) respectif sur chaque corps d'électrode (20),
d) le cas échéant, appliquer une résine époxy sur l'emplacement de connexion,
e) couler un moule de l'ensemble d'électrodes (1) à partir du matériau de support (6),
f) incorporer les corps d'électrode (20) et, le cas échéant, les conducteurs électriques (4) et, le cas échéant, l'élément de renforcement (8),
g) raccorder un conducteur électrique (4) à la partie de mise en contact (9) de l'ensemble d'électrodes (1).

14. Procédé de fabrication d'un ensemble d'électrodes (1) pour la stimulation électrique du tissu d'un être vivant selon l'une des revendications précédentes, **caractérisé par** les étapes suivantes consistant à :
a) fournir un matériau de support (6),
b) produire un ou plusieurs corps d'électrode (20) sur le matériau de support (6) par un processus de dépôt en phase vapeur et/ou par dépôt galvanique,
c) le cas échéant, appliquer un revêtement de surface et/ou une structuration de surface sur au moins une partie de la surface de l'électrode,
d) le cas échéant, incorporer le matériau de support (6) dans un autre matériau de support isolant.
